(19) Europäisches Patentamt
European Patent Office
Office européen des brevets

(11) **EP 4 129 294 A1**

(12) **EUROPEAN PATENT APPLICATION**
published in accordance with Art. 153(4) EPC


(43) Date of publication:
**08.02.2023 Bulletin 2023/06**

(21) Application number: **21781213.0**

(22) Date of filing: **02.04.2021**

(51) International Patent Classification (IPC):
***A61K 31/53*** *(1974.07)* ***A61P 25/04*** *(2000.01)*
***A61P 29/02*** *(2000.01)* ***A61P 43/00*** *(2000.01)*

(52) Cooperative Patent Classification (CPC):
**A61K 31/53; A61P 25/04; A61P 29/02; A61P 43/00**

(86) International application number:
**PCT/JP2021/014261**

(87) International publication number:
**WO 2021/201261 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.04.2020 JP 2020067146**



(71) Applicant: **Shionogi & Co., Ltd**
**Osaka-shi, Osaka 541-0045 (JP)**

(72) Inventors:
- **KAMEYAMA Takayuki**
  **Osaka-shi, Osaka 5410045 (JP)**
- **KAI Hiroyuki**
  **Toyonaka-shi, Osaka 561-0825 (JP)**

(74) Representative: **Vossius & Partner**
**Patentanwälte Rechtsanwälte mbB**
**Siebertstraße 3**
**81675 München (DE)**


(54) **PAIN TREATMENT DRUG**


(57) The present invention relates to a pharmaceutical composition comprising a $P2X_3$ and/or $P2X_{2/3}$ receptor antagonist for the treatment of neuropathic pain, nociceptive pain or visceral pain, as well as interstitial cystitis and/or bladder pain syndrome.

It is a pharmaceutical composition for treating and/or preventing neuropathic pain, nociceptive pain, or visceral pain, as well as interstitial cystitis and/or bladder pain syndrome, comprising a compound represented by Formula (I):

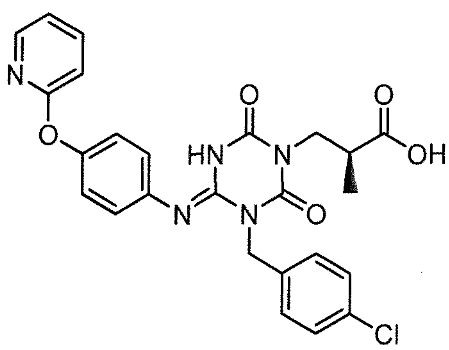

(I)

or a pharmaceutically acceptable salt thereof.


Fig. 1

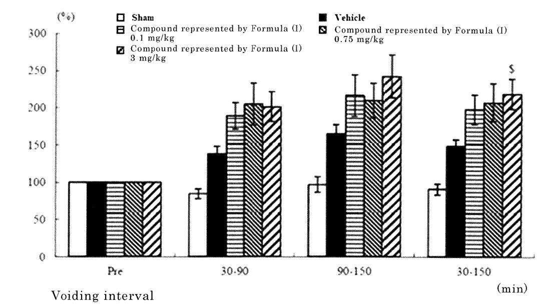


EP 4 129 294 A1

## Description

[TECHNICAL FIELD]

The present invention relates to the treatment of diseases involving P2X receptors, particularly $P2X_3$ and/or $P2X_{2/3}$ receptors. In particular, the present invention relates to a pharmaceutical composition comprising a $P2X_3$ and/or $P2X_{2/3}$ receptor antagonist for the treatment of neuropathic pain, nociceptive pain or visceral pain, as well as interstitial cystitis and/or bladder pain syndrome.

[BACKGROUND ART]

Adenosine triphosphate (ATP) is known as an intracellular energy source and a phosphorylated substrate. On the other hand, it is also known to act as an extracellular information transmission substance. It is known that ATP is released from a cell in response to various stimulation such as cellular injury, inflammation, nociceptive stimulus, reduced blood oxygen level, and it is also known that ATP is released together with another neurotransmitter from a primary sensory nerve terminal to outside a cell. ATP thus released to outside a cell mediates various extracellular information transmission through an ATP receptor (Non-patent Documents 1, 2).
ATP receptor is categorized into ionotropic P2X family and G protein-coupled P2Y family. For P2X receptor family, seven subtypes have been reported, and a member of this family forms a homo-trimeric structure or a hetero-trimeric structure together with another member of a subtype of this P2X receptor family and functions as a non-selective cation channel (Non-patent Document 3).
It is already known that ATP causes pain, and it has been indicated that local administration of the same to humans and animals causes pain (Non-patent Document 4). Further, studies using knockout and knockdown techniques of $P2X_3$ have shown that the $P2X_3$ receptor is involved in the transmission of chronic pain. The $P2X_3$ receptor is expressed in a peripheral sensory nerve-specific manner, forming a homo-complex or a hetero-complex with $P2X_2$ ($P2X_{2/3}$). (Non-patent Document 5)
Subsequently, a compound designated as A-317491 was reported as an antagonist specific for the $P2X_3$ and $P2X_{2/3}$ receptors. A-317491 is a trisubstituted-N-[(1S)-1,2,3,4-tetiahydro-1-naphthalenyl]benzamide derivative (Patent Document 1) represented by the following formula:

[Chemical Formula 1]

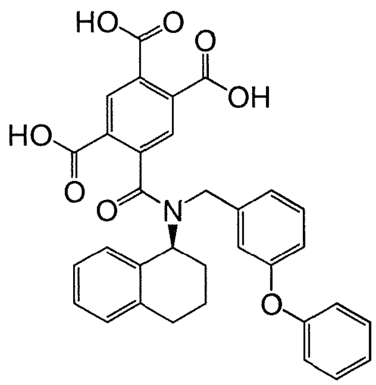

and this compound has been reported to exhibit an antagonistic activity against $P2X_3$ and $P2X_{2/3}$ receptors and exhibit an analgesic effect in a rat neuropathic pain model and an inflammatory pain model (Non-patent Documents 6 and 7). This indicates that pain sensation is transmitted via the $P2X_3$ or $P2X_{2/3}$ receptor and that a compound having P2Xs or $P2X_{2/3}$ receptor antagonism is useful as an analgesic.
On the other hand, Non-patent Document 8 reports that the $P2X_3$ receptor is expressed in nerves projecting to the knee joint, and reports that AF-219 (MK-7264, Gefapixant), a P2X3 and $P2X_{2/3}$ antagonist, exhibits an analgesic effect in a rat knee osteoarthritis model.
In addition, a large number of clinical trial protocols for AF-219 (MK-7264, Gefapixant) and their results are recorded on ClinicalTrials.gov. For example, the results of a trial in which 171 knee osteoarthritis patients were randomly assigned to a twice daily oral administration group of a placebo or 300 mg of AF-219 to confirm the therapeutic effect every week up to 4 weeks have been published under the identification number NCT01554579. The numeric pain rating

scale (NPRS) before administration was 6.8, whereas the NPRS of the Gefapixant administration group was reduced to 4.9 after 4 weeks administration (the NPRS of the Procebo group was reduced from 6.7 to 5.3).

In addition, the results of a trial in which 74 patients of interstitial cystitis/bladder pain syndrome were randomly assigned to a twice daily oral administration group of a placebo or AF-219 (gradually increased from 50 mg to 300 mg) to confirm the therapeutic effect after 4 weeks have been published under the identification number NCT01569438. This reports the analgesic effect of AF-219. The NPRS before administration was 6.2, whereas the NPRS of the Gefapixant administration group was reduced to 3.3 after administration (the NPRS of the Procebo group was reduced from 6.4 to 4.5).

Furthermore, since the $P2X_3$ receptor is expressed in nerves projecting to the pancreas and A-317491 as a $P2X_3$ receptor inhibitor suppresses pain associated with pancreatitis, it is suggested that the $P2X_3$ receptor is involved in the pain associated with pancreatitis (Non-patent Document 9).

In addition, since the $P2X_3$ receptor is expressed in nerves in the endometrium (Non-patent Document 10), and A-317491 as a $P2X_3$ receptor inhibitor suppresses pain, it is suggested that the $P2X_3$ receptor is involved in the pain associated with endometriosis (Non-patent Document 11). This report suggests that compounds having $P2X_3$ receptor antagonism may be useful in the treatment of pain associated with endometriosis.

Patent Documents 2 to 5 disclose a large number of compounds exhibiting $P2X_3$ and/or $P2X_{2/3}$ receptor antagonist activity and describes them as being effective in treating, for example, pain such as neuropathic pain. In addition, Patent Documents 3 to 5 describes a compound whose analgesic effect has been confirmed by the pharmacological studies using the Seltzer model, but does not describe the analgesic effect on neuropathic pain (for example, diabetic neuropathic pain), nociceptive pain (for example, inflammatory pain, knee osteoarthritis pain, cancer pain), and visceral pain (for example, cystitis pain, cancer pain, pain associated with endometriosis). Furthermore, Patent Document 3 describes drug efficacy evaluation using a rat cystitis model that indicates an improvement in voiding function (voiding interval and amount of voided urine), which however does not mention an analgesic effect on pain associated with cystitis. In addition, Patent Document 6 does not describe an effect on neuropathic pain, nociceptive pain or visceral pain, and interstitial cystitis and/or bladder pain syndrome.

[PRIOR ART REFERENCES]

[Patent Document]

[Patent Document 1] International Publication WO 02/094767
[Patent Document 2] International Publication WO 2010/092966
[Patent Document 3] International Publication WO 2012/020749
[Patent Document 4] International Publication WO 2013/089212
[Patent Document 5] International Publication WO 2014/200078
[Patent Document 6] International Publication WO 2020/071530

[Non-patent Document]

[Non-patent Document 1] J. Physiology 2003, Vol. 554, No. 2, pp. 301-308
[Non-patent Document 2] J. Physiology 2003, Vol. 553, No. 3, pp. 683-694
[Non-patent Document 3] Pflungers Arch Eur J physiol 2006, pp. 452, 513-537
[Non-patent Document 4] Neuroscientist 2005, Vol. 11, pp. 345-356
[Non-patent Document 5] Mol Neurobiol 2007; 36: pp. 165-83
[Non-patent Document 6] Expert Opin.Ther.Patens 2006 Vol. 16, No. 8, pp. 1113-1127
[Non-patent Document 7] PNAS 2002, Vol. 99, No. 26, pp. 17179-17184
[Non-patent Document 8] Purinergic Signal. 2012 vol. 8, pp. 3-26
[Non-patent Document 9] Am J Physiol Gastrointest Liver Physiol 2015, Vol. 308, pp. 710-719
[Non-patent Document 10] PLoS ONE 2017, Vol. 12, No. 9
[Non-patent Document 11] International Journal of Nanomedicine 2017, Volume 12, 8171-8183

[SUMMARY OF THE INVENTION]

[PROBLEMS TO BE SOLVED BY THE INVENTION]

It is an object of the present invention to provide a pharmaceutical composition with excellent $P2X_3$ receptor antagonist activity to be used for pain therapy, and therapy for interstitial cystitis and/or bladder pain syndrome.

[MEANS FOR SOLVING THE PROBLEM]

As a result of intensive studies to solve the above problems, the present inventors have found that among $P2X_3$ and/or $P2X_{2/3}$ receptor antagonists described in Patent Document 5, a specific compound has excellent $P2X_3$ receptor antagonist activity, and has an analgesic effect on neuropathic pain (for example, diabetic neuropathic pain), nociceptive pain (for example, inflammatory pain, knee osteoarthritis pain, cancer pain), and visceral pain (for example, cystitis pain, cancer pain, pain associated with endometriosis), as well as that the voiding interval is prolonged and the amount of voided urine is increased accordingly. The present inventors thereby completed the present invention.

The present invention also relates to the following (1) to (8), (8-a) to (8-z), (9), (9-a) to (9-d), (10) to (17), (17-a) to (17-z), (18), (18-a) to (18-d), (19) to (26), (26-a) to (26-z), (27), (27-a) to (27-d), (28) to (35), (35-a) to (35-z), (36), and (36-a) to (36-d).

(1) A pharmaceutical composition for treating, alleviating, and/or preventing neuropathic pain, nociceptive pain, or visceral pain, comprising a compound represented by Formula (I):

[Chemical Formula 2]

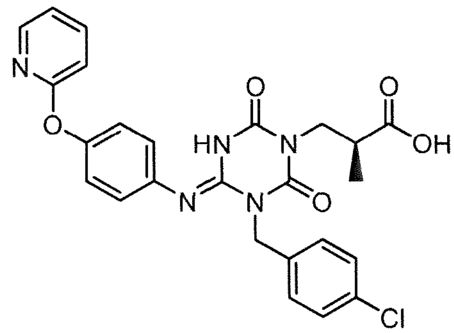

(I)

or a pharmaceutically acceptable salt thereof.

(2) The pharmaceutical composition according to (1), wherein the neuropathic pain is peripheral neuropathic pain.

(3) The pharmaceutical composition according to (1) or (2), wherein the neuropathic pain is diabetic neuropathic pain, postoperative neuropathic pain, or cancer pain.

(4) The pharmaceutical composition according to any one of (1) to (3), wherein the neuropathic pain is diabetic neuropathic pain.

(5) The pharmaceutical composition according to (1), wherein the nociceptive pain is inflammatory pain, knee osteoarthritis pain, or cancer pain.

(6) The pharmaceutical composition according to (1), wherein the visceral pain is cystitis pain, cancer pain, or pain associated with endometriosis.

(7) The pharmaceutical composition according to (1) or (6), wherein the visceral pain is cystitis pain.

(8) The pharmaceutical composition according to any one of (1) to (7), wherein a daily dose of an active ingredient of the pharmaceutical composition is 10 mg to 450 mg.

(8-a) The pharmaceutical composition according to any one of (1) to (8), wherein a daily dose of an active ingredient of the pharmaceutical composition is 10 mg to 300 mg.

(8-b) The pharmaceutical composition according to any one of (1) to (8), wherein a daily dose of an active ingredient of the pharmaceutical composition is 20 mg to 300 mg.

(8-c) The pharmaceutical composition according to any one of (1) to (8), wherein a daily dose of an active ingredient of the pharmaceutical composition is 30 mg to 300 mg.

(8-d) The pharmaceutical composition according to any one of (1) to (8), wherein a daily dose of an active ingredient of the pharmaceutical composition is 50 mg to 300 mg.

(8-e) The pharmaceutical composition according to any one of (1) to (8), wherein a daily dose of an active

ingredient of the pharmaceutical composition is 70 mg to 300 mg.
(8-f) The pharmaceutical composition according to any one of (1) to (8), wherein a daily dose of an active ingredient of the pharmaceutical composition is 100 mg to 300 mg.
(8-g) The pharmaceutical composition according to any one of (1) to (8), wherein a daily dose of an active ingredient of the pharmaceutical composition is 150 mg to 300 mg.
(8-h) The pharmaceutical composition according to any one of (1) to (8), wherein a daily dose of an active ingredient of the pharmaceutical composition is 200 mg to 300 mg.
(8-i) The pharmaceutical composition according to any one of (1) to (8), wherein a daily dose of an active ingredient of the pharmaceutical composition is 10 mg to 150 mg.
(8-j) The pharmaceutical composition according to any one of (1) to (8), wherein a daily dose of an active ingredient of the pharmaceutical composition is 20 mg to 150 mg.
(8-k) The pharmaceutical composition according to any one of (1) to (8), wherein a daily dose of an active ingredient of the pharmaceutical composition is 30 mg to 150 mg.
(8-1) The pharmaceutical composition according to any one of (1) to (8), wherein a daily dose of an active ingredient of the pharmaceutical composition is 50 mg to 150 mg.
(8-m) The pharmaceutical composition according to any one of (1) to (8), wherein a daily dose of an active ingredient of the pharmaceutical composition is 70 mg to 150 mg.
(8-n) The pharmaceutical composition according to any one of (1) to (8), wherein a daily dose of an active ingredient of the pharmaceutical composition is 100 mg to 150 mg.
(8-o) The pharmaceutical composition according to any one of (1) to (8), wherein a daily dose of an active ingredient of the pharmaceutical composition is 50 mg to 200 mg.
(8-p) The pharmaceutical composition according to any one of (1) to (8), wherein a daily dose of an active ingredient of the pharmaceutical composition is 100 mg to 200 mg.
(8-q) The pharmaceutical composition according to any one of (1) to (8), wherein a daily dose of an active ingredient of the pharmaceutical composition is 150 mg to 200 mg.
(8-r) The pharmaceutical composition according to any one of (1) to (8), wherein a daily dose of an active ingredient of the pharmaceutical composition is 300 mg.
(8-s) The pharmaceutical composition according to any one of (1) to (8), wherein a daily dose of an active ingredient of the pharmaceutical composition is 200 mg.
(8-t) The pharmaceutical composition according to any one of (1) to (8), wherein a daily dose of an active ingredient of the pharmaceutical composition is 150 mg.
(8-u) The pharmaceutical composition according to any one of (1) to (8), wherein a daily dose of an active ingredient of the pharmaceutical composition is 100 mg.
(8-v) The pharmaceutical composition according to any one of (1) to (8), wherein a daily dose of an active ingredient of the pharmaceutical composition is 70 mg.
(8-w) The pharmaceutical composition according to any one of (1) to (8), wherein a daily dose of an active ingredient of the pharmaceutical composition is 50 mg.
(8-x) The pharmaceutical composition according to any one of (1) to (8), wherein a daily dose of an active ingredient of the pharmaceutical composition is 30 mg.
(8-y) The pharmaceutical composition according to any one of (1) to (8), wherein a daily dose of an active ingredient of the pharmaceutical composition is 20 mg.
(8-z) The pharmaceutical composition according to any one of (1) to (8), wherein a daily dose of an active ingredient of the pharmaceutical composition is 10 mg.

(9) The pharmaceutical composition according to any one of (1) to (8) and (8-a) to (8-z), to be administered once daily.

(9-a) The pharmaceutical composition according to any one of (1) to (8), (8-a) to (8-z), and (9), to be administered once daily after a meal.
(9-b) The pharmaceutical composition according to any one of (1) to (8), (8-a) to (8-z), and (9), to be administered once daily at bedtime.
(9-c) The pharmaceutical composition according to any one of (1) to (8), (8-a) to (8-z), and (9), to be administered once daily before a meal.
(9-d) The pharmaceutical composition according to any one of (1) to (8), (8-a) to (8-z), and (9), to be administered once daily between meals.

(10) A method for treating, alleviating, and/or preventing neuropathic pain, nociceptive pain, or visceral pain, the method comprising the step of administering an effective amount of a compound represented by Formula (I):

[Chemical Formula 3]

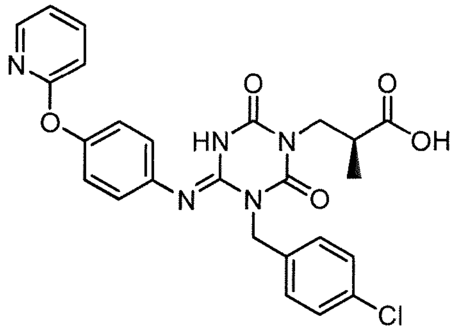

(I)

or a pharmaceutically acceptable salt thereof to an individual in need of treatment, alleviation, and/or prevention of neuropathic pain, nociceptive pain, or visceral pain.

(11) The treating, alleviating, and/or preventing method according to (10), wherein the neuropathic pain is peripheral neuropathic pain.

(12) The treating, alleviating, and/or preventing method according to (10) or (11), wherein the neuropathic pain is diabetic neuropathic pain, postoperative neuropathic pain, or cancer pain.

(13) The treating, alleviating, and/or preventing method according to any one of (10) to (12), wherein the neuropathic pain is diabetic neuropathic pain.

(14) The treating, alleviating, and/or preventing method according to (10), wherein the nociceptive pain is inflammatory pain, knee osteoarthritis pain, or cancer pain.

(15) The treating, alleviating, and/or preventing method according to (10), wherein the visceral pain is cystitis pain, cancer pain, or pain associated with endometriosis.

(16) The treating, alleviating, and/or preventing method according to (10) or (15), wherein the visceral pain is cystitis pain.

(17) The treating, alleviating, and/or preventing method according to any one of (10) to (16), wherein a daily dose of an active ingredient is 10 mg to 450 mg.

(17-a) The treating, alleviating, and/or preventing method according to any one of (10) to (17), wherein a daily dose of an active ingredient is 10 mg to 300 mg.

(17-b) The treating, alleviating, and/or preventing method according to any one of (10) to (17), wherein a daily dose of an active ingredient is 20 mg to 300 mg.

(17-c) The treating, alleviating, and/or preventing method according to any one of (10) to (17), wherein a daily dose of an active ingredient is 30 mg to 300 mg.

(17-d) The treating, alleviating, and/or preventing method according to any one of (10) to (17), wherein a daily dose of an active ingredient is 50 mg to 300 mg.

(17-e) The treating, alleviating, and/or preventing method according to any one of (10) to (17), wherein a daily dose of an active ingredient is 70 mg to 300 mg.

(17-f) The treating, alleviating, and/or preventing method according to any one of (10) to (17), wherein a daily dose of an active ingredient is 100 mg to 300 mg.

(17-g) The treating, alleviating, and/or preventing method according to any one of (10) to (17), wherein a daily dose of an active ingredient is 150 mg to 300 mg.

(17-h) The treating, alleviating, and/or preventing method according to any one of (10) to (17), wherein a daily dose of an active ingredient is 200 mg to 300 mg.

(17-i) The treating, alleviating, and/or preventing method according to any one of (10) to (17), wherein a daily dose of an active ingredient is 10 mg to 150 mg.

(17-j) The treating, alleviating, and/or preventing method according to any one of (10) to (17), wherein a daily dose of an active ingredient is 20 mg to 150 mg.

(17-k) The treating, alleviating, and/or preventing method according to any one of (10) to (17), wherein a daily dose of an active ingredient is 30 mg to 150 mg.

(17-l) The treating, alleviating, and/or preventing method according to any one of (10) to (17), wherein a daily dose of an active ingredient is 50 mg to 150 mg.

(17-m) The treating, alleviating, and/or preventing method according to any one of (10) to (17), wherein a daily dose of an active ingredient is 70 mg to 150 mg.

(17-n) The treating, alleviating, and/or preventing method according to any one of (10) to (17), wherein a daily dose of an active ingredient is 100 mg to 150 mg.

(17-o) The treating, alleviating, and/or preventing method according to any one of (10) to (17), wherein a daily dose of an active ingredient is 50 mg to 200 mg.
(17-p) The treating, alleviating, and/or preventing method according to any one of (10) to (17), wherein a daily dose of an active ingredient is 100 mg to 200 mg.
(17-q) The treating, alleviating, and/or preventing method according to any one of (10) to (17), wherein a daily dose of an active ingredient is 150 mg to 200 mg.
(17-r) The treating, alleviating, and/or preventing method according to any one of (10) to (17), wherein a daily dose of an active ingredient is 300 mg.
(17-s) The treating, alleviating, and/or preventing method according to any one of (10) to (17), wherein a daily dose of an active ingredient is 200 mg.
(17-t) The treating, alleviating, and/or preventing method according to any one of (10) to (17), wherein a daily dose of an active ingredient is 150 mg.
(17-u) The treating, alleviating, and/or preventing method according to any one of (10) to (17), wherein a daily dose of an active ingredient is 100 mg.
(17-v) The treating, alleviating, and/or preventing method according to any one of (10) to (17), wherein a daily dose of an active ingredient is 70 mg.
(17-w) The treating, alleviating, and/or preventing method according to any one of (10) to (17), wherein a daily dose of an active ingredient is 50 mg.
(17-x) The treating, alleviating, and/or preventing method according to any one of (10) to (17), wherein a daily dose of an active ingredient is 30 mg.
(17-y) The treating, alleviating, and/or preventing method according to any one of (10) to (17), wherein a daily dose of an active ingredient is 20 mg.
(17-z) The treating, alleviating, and/or preventing method according to any one of (10) to (17), wherein a daily dose of an active ingredient is 10 mg.

(18) The treating, alleviating, and/or preventing method according to any one of (10) to (17) and (17-a) to (17-z), wherein the administering step is performed once daily.

(18-a) The treating, alleviating, and/or preventing method according to any one of (10) to (17), (17-a) to (17-z), and (18), wherein the administering step is performed once daily after a meal.
(18-b) The treating, alleviating, and/or preventing method according to any one of (10) to (17), (17-a) to (17-z), and (18), wherein the administering step is performed once daily at bedtime.
(18-c) The treating, alleviating, and/or preventing method according to any one of (10) to (17), (17-a) to (17-z), and (18), wherein the administering step is performed once daily before a meal.
(18-d) The treating, alleviating, and/or preventing method according to any one of (10) to (17), (17-a) to (17-z), and (18), wherein the administering step is performed once daily between meals.

(19) Use of a compound represented by Formula (I):

[Chemical Formula 4]

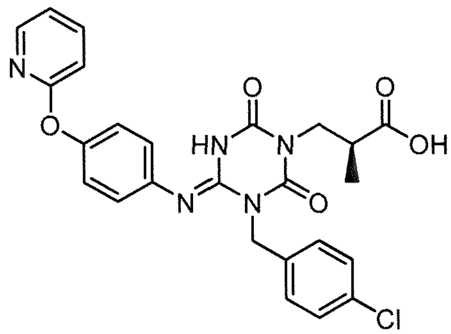

(I)

or a pharmaceutically acceptable salt thereof for manufacture of a medicament for treating, alleviating, and/or preventing neuropathic pain, nociceptive pain, or visceral pain.
(20) The use according to (19), wherein the neuropathic pain is peripheral neuropathic pain.
(21) The use according to (19) or (20), wherein the neuropathic pain is diabetic neuropathic pain, postoperative neuropathic pain, or cancer pain.
(22) The use according to any one of (19) to (21), wherein the neuropathic pain is diabetic neuropathic pain.

(23) The use according to (19), wherein the nociceptive pain is inflammatory pain, knee osteoarthritis pain, or cancer pain.
(24) The use according to (19), wherein the visceral pain is cystitis pain, cancer pain, or pain associated with endometriosis.
(25) The use according to (19) or (24), wherein the visceral pain is cystitis pain.
(26) The use according to any one of (19) to (25), wherein a daily dose of an active ingredient is 10 mg to 450 mg.

(26-a) The use according to any one of (19) to (26), wherein a daily dose of an active ingredient is 10 mg to 300 mg.
(26-b) The use according to any one of (19) to (26), wherein a daily dose of an active ingredient is 20 mg to 300 mg.
(26-c) The use according to any one of (19) to (26), wherein a daily dose of an active ingredient is 30 mg to 300 mg.
(26-d) The use according to any one of (19) to (26), wherein a daily dose of an active ingredient is 50 mg to 300 mg.
(26-e) The use according to any one of (19) to (26), wherein a daily dose of an active ingredient is 70 mg to 300 mg.
(26-f) The use according to any one of (19) to (26), wherein a daily dose of an active ingredient is 100 mg to 300 mg.
(26-g) The use according to any one of (19) to (26), wherein a daily dose of an active ingredient is 150 mg to 300 mg.
(26-h) The use according to any one of (19) to (26), wherein a daily dose of an active ingredient is 200 mg to 300 mg.
(26-i) The use according to any one of (19) to (26), wherein a daily dose of an active ingredient is 10 mg to 150 mg.
(26-j) The use according to any one of (19) to (26), wherein a daily dose of an active ingredient is 20 mg to 150 mg.
(26-k) The use according to any one of (19) to (26), wherein a daily dose of an active ingredient is 30 mg to 150 mg.
(26-l) The use according to any one of (19) to (26), wherein a daily dose of an active ingredient is 50 mg to 150 mg.
(26-m) The use according to any one of (19) to (26), wherein a daily dose of an active ingredient is 70 mg to 150 mg.
(26-n) The use according to any one of (19) to (26), wherein a daily dose of an active ingredient is 100 mg to 150 mg.
(26-o) The use according to any one of (19) to (26), wherein a daily dose of an active ingredient is 50 mg to 200 mg.
(26-p) The use according to any one of (19) to (26), wherein a daily dose of an active ingredient is 100 mg to 200 mg.
(26-q) The use according to any one of (19) to (26), wherein a daily dose of an active ingredient is 150 mg to 200 mg.
(26-r) The use according to any one of (19) to (26), wherein a daily dose of an active ingredient is 300 mg.
(26-s) The use according to any one of (19) to (26), wherein a daily dose of an active ingredient is 200 mg.
(26-t) The use according to any one of (19) to (26), wherein a daily dose of an active ingredient is 150 mg.
(26-u) The use according to any one of (19) to (26), wherein a daily dose of an active ingredient is 100 mg.
(26-v) The use according to any one of (19) to (26), wherein a daily dose of an active ingredient is 70 mg.
(26-w) The use according to any one of (19) to (26), wherein a daily dose of an active ingredient is 50 mg.
(26-x) The use according to any one of (19) to (26), wherein a daily dose of an active ingredient is 30 mg.
(26-y) The use according to any one of (19) to (26), wherein a daily dose of an active ingredient is 20 mg.
(26-z) The use according to any one of (19) to (26), wherein a daily dose of an active ingredient is 10 mg.

(27) The use according to any one of (19) to (26) and (26-a) to (26-z), wherein the medicament is to be administered once daily.

(27-a) The use according to any one of (19) to (26), (26-a) to (26-z), and (27), wherein the medicament is to be administered once daily after a meal.

(27-b) The use according to any one of (19) to (26), (26-a) to (26-z), and (27), wherein the medicament is to be administered once daily at bedtime.

(27-c) The use according to any one of (19) to (26), (26-a) to (26-z), and (27), wherein the medicament is to be administered once daily before a meal.

(27-d) The use according to any one of (19) to (26), (26-a) to (26-z), and (27), wherein the compound or the salt is to be administered once daily between meals.

(28) A compound represented by Formula (I):

[Chemical Formula 5]

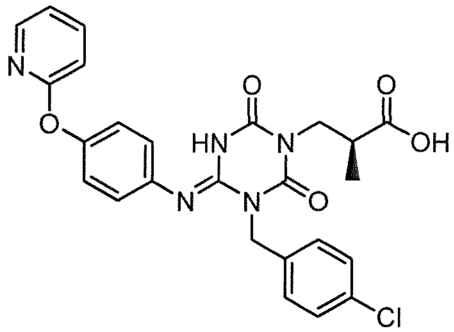

(I)

or a pharmaceutically acceptable salt thereof to be used for treating, alleviating, and/or preventing neuropathic pain, nociceptive pain, or visceral pain.

(29) The compound according to (28) or a pharmaceutically acceptable salt thereof, wherein the neuropathic pain is peripheral neuropathic pain.

(30) The compound according to (28) or (29) or a pharmaceutically acceptable salt thereof, wherein the neuropathic pain is diabetic neuropathic pain, postoperative neuropathic pain, or cancer pain.

(31) The compound according to any one of (28) to (30) or a pharmaceutically acceptable salt thereof, wherein the neuropathic pain is diabetic neuropathic pain.

(32) The compound according to (28) or a pharmaceutically acceptable salt thereof, wherein the nociceptive pain is inflammatory pain, knee osteoarthritis pain, or cancer pain.

(33) The compound according to (28) or a pharmaceutically acceptable salt thereof, wherein the visceral pain is cystitis pain, cancer pain, or pain associated with endometriosis.

(34) The compound according to (28) or (33) or a pharmaceutically acceptable salt thereof, wherein the visceral pain is cystitis pain

(35) The compound according to any one of (28) to (34) or a pharmaceutically acceptable salt thereof, wherein a daily dose of an active ingredient is 10 mg to 450 mg.

(35-a) The compound according to any one of (28) to (35) or a pharmaceutically acceptable salt thereof, wherein a daily dose of an active ingredient is 10 mg to 300 mg.

(35-b) The compound according to any one of (28) to (35) or a pharmaceutically acceptable salt thereof, wherein a daily dose of an active ingredient is 20 mg to 300 mg.

(35-c) The compound according to any one of (28) to (35) or a pharmaceutically acceptable salt thereof, wherein a daily dose of an active ingredient is 30 mg to 300 mg.

(35-d) The compound according to any one of (28) to (35) or a pharmaceutically acceptable salt thereof, wherein a daily dose of an active ingredient is 50 mg to 300 mg.

(35-e) The compound according to any one of (28) to (35) or a pharmaceutically acceptable salt thereof, wherein a daily dose of an active ingredient is 70 mg to 300 mg.

(35-f) The compound according to any one of (28) to (35) or a pharmaceutically acceptable salt thereof, wherein a daily dose of an active ingredient is 100 mg to 300 mg.

(35-g) The compound according to any one of (28) to (35) or a pharmaceutically acceptable salt thereof, wherein a daily dose of an active ingredient is 150 mg to 300 mg.

(35-h) The compound according to any one of (28) to (35) or a pharmaceutically acceptable salt thereof, wherein a daily dose of an active ingredient is 200 mg to 300 mg.

(35-i) The compound according to any one of (28) to (35) or a pharmaceutically acceptable salt thereof, wherein a daily dose of an active ingredient is 10 mg to 150 mg.

(35-j) The compound according to any one of (28) to (35) or a pharmaceutically acceptable salt thereof, wherein a daily dose of an active ingredient is 20 mg to 150 mg.

(35-k) The compound according to any one of (28) to (35) or a pharmaceutically acceptable salt thereof, wherein a daily dose of an active ingredient is 30 mg to 150 mg.

(35-l) The compound according to any one of (28) to (35) or a pharmaceutically acceptable salt thereof, wherein a daily dose of an active ingredient is 50 mg to 150 mg.

(35-m) The compound according to any one of (28) to (35) or a pharmaceutically acceptable salt thereof, wherein a daily dose of an active ingredient is 70 mg to 150 mg.

(35-n) The compound according to any one of (28) to (35) or a pharmaceutically acceptable salt thereof, wherein a daily dose of an active ingredient is 100 mg to 150 mg.

(35-o) The compound according to any one of (28) to (35) or a pharmaceutically acceptable salt thereof, wherein

a daily dose of an active ingredient is 50 mg to 200 mg.
(35-p) The compound according to any one of (28) to (35) or a pharmaceutically acceptable salt thereof, wherein a daily dose of an active ingredient is 100 mg to 200 mg.
(35-q) The compound according to any one of (28) to (35) or a pharmaceutically acceptable salt thereof, wherein a daily dose of an active ingredient is 150 mg to 200 mg.
(35-r) The compound according to any one of (28) to (35) or a pharmaceutically acceptable salt thereof, wherein a daily dose of an active ingredient is 300 mg.
(35-s) The compound according to any one of (28) to (35) or a pharmaceutically acceptable salt thereof, wherein a daily dose of an active ingredient is 200 mg.
(35-t) The compound according to any one of (28) to (35) or a pharmaceutically acceptable salt thereof, wherein a daily dose of an active ingredient is 150 mg.
(35-u) The compound according to any one of (28) to (35) or a pharmaceutically acceptable salt thereof, wherein a daily dose of an active ingredient is 100 mg.
(35-v) The compound according to any one of (28) to (35) or a pharmaceutically acceptable salt thereof, wherein a daily dose of an active ingredient is 70 mg.
(35-w) The compound according to any one of (28) to (35) or a pharmaceutically acceptable salt thereof, wherein a daily dose of an active ingredient is 50 mg.
(35-x) The compound according to any one of (28) to (35) or a pharmaceutically acceptable salt thereof, wherein a daily dose of an active ingredient is 30 mg.
(35-y) The compound according to any one of (28) to (35) or a pharmaceutically acceptable salt thereof, wherein a daily dose of an active ingredient is 20 mg.
(35-z) The compound according to any one of (28) to (35) or a pharmaceutically acceptable salt thereof, wherein a daily dose of an active ingredient is 10 mg.

(36) The compound according to any one of (28) to (35) and (35-a) to (35-z) or a pharmaceutically acceptable salt thereof, to be administered once daily.

(36-a) The compound according to any one of (28) to (35), (35-a) to (35-z), and (36) or a pharmaceutically acceptable salt thereof, to be administered once daily after a meal.
(36-b) The compound according to any one of (28) to (35), (35-a) to (35-z), and (36) or a pharmaceutically acceptable salt thereof, to be administered once daily at bedtime.
(36-c) The compound according to any one of (28) to (35), (35-a) to (35-z), and (36) or a pharmaceutically acceptable salt thereof, to be administered once daily before a meal.
(36-d) The compound according to any one of (28) to (35), (35-a) to (35-z), and (36) or a pharmaceutically acceptable salt thereof, to be administered once daily between meals.

The present invention also relates to the following (101), (102), (102-a) to (102-z), (103), (103-a) to (103-d), (104), (105), (105-a) to (105-z), (106), (106-a) to (106-d), (107), (108), (108-a) to (108-z), (109), (109-a) to (109-d), (110), (111), (111-a) to (111-z), (112), and (112-a) to (112-d).

(101) A pharmaceutical composition for treating, alleviating, and/or preventing interstitial cystitis and/or bladder pain syndrome, the pharmaceutical composition containing a compound represented by Formula (I):

[Chemical Formula 6]

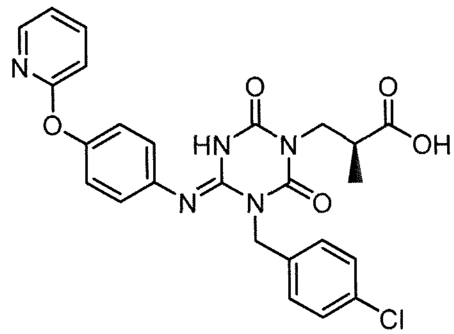

(I)

or a pharmaceutically acceptable salt thereof.
(102) The pharmaceutical composition according to (101), wherein a daily dose of an active ingredient of the pharmaceutical composition is 10 mg to 450 mg.

(102-a) The pharmaceutical composition according to (101) or (102), wherein a daily dose of an active ingredient of the pharmaceutical composition is 10 mg to 300 mg.
(102-b) The pharmaceutical composition according to (101) or (102), wherein a daily dose of an active ingredient of the pharmaceutical composition is 20 mg to 300 mg.
(102-c) The pharmaceutical composition according to (101) or (102), wherein a daily dose of an active ingredient of the pharmaceutical composition is 30 mg to 300 mg.
(102-d) The pharmaceutical composition according to (101) or (102), wherein a daily dose of an active ingredient of the pharmaceutical composition is 50 mg to 300 mg.
(102-e) The pharmaceutical composition according to (101) or (102), wherein a daily dose of an active ingredient of the pharmaceutical composition is 70 mg to 300 mg.
(102-f) The pharmaceutical composition according to (101) or (102), wherein a daily dose of an active ingredient of the pharmaceutical composition is 100 mg to 300 mg.
(102-g) The pharmaceutical composition according to (101) or (102), wherein a daily dose of an active ingredient of the pharmaceutical composition is 150 mg to 300 mg.
(102-h) The pharmaceutical composition according to (101) or (102), wherein a daily dose of an active ingredient of the pharmaceutical composition is 200 mg to 300 mg.
(102-i) The pharmaceutical composition according to (101) or (102), wherein a daily dose of an active ingredient of the pharmaceutical composition is 10 mg to 150 mg.
(102-j) The pharmaceutical composition according to (101) or (102), wherein a daily dose of an active ingredient of the pharmaceutical composition is 20 mg to 150 mg.
(102-k) The pharmaceutical composition according to (101) or (102), wherein a daily dose of an active ingredient of the pharmaceutical composition is 30 mg to 150 mg.
(102-l) The pharmaceutical composition according to (101) or (102), wherein a daily dose of an active ingredient of the pharmaceutical composition is 50 mg to 150 mg.
(102-m) The pharmaceutical composition according to (101) or (102), wherein a daily dose of an active ingredient of the pharmaceutical composition is 70 mg to 150 mg.
(102-n) The pharmaceutical composition according to (101) or (102), wherein a daily dose of an active ingredient of the pharmaceutical composition is 100 mg to 150 mg.
(102-o) The pharmaceutical composition according to (101) or (102), wherein a daily dose of an active ingredient of the pharmaceutical composition is 50 mg to 200 mg.
(102-p) The pharmaceutical composition according to (101) or (102), wherein a daily dose of an active ingredient of the pharmaceutical composition is 100 mg to 200 mg.
(102-q) The pharmaceutical composition according to (101) or (102), wherein a daily dose of an active ingredient of the pharmaceutical composition is 150 mg to 200 mg.
(102-r) The pharmaceutical composition according to (101) or (102), wherein a daily dose of an active ingredient of the pharmaceutical composition is 300 mg.
(102-s) The pharmaceutical composition according to (101) or (102), wherein a daily dose of an active ingredient of the pharmaceutical composition is 200 mg.
(102-t) The pharmaceutical composition according to (101) or (102), wherein a daily dose of an active ingredient of the pharmaceutical composition is 150 mg.
(102-u) The pharmaceutical composition according to (101) or (102), wherein a daily dose of an active ingredient of the pharmaceutical composition is 100 mg.
(102-v) The pharmaceutical composition according to (101) or (102), wherein a daily dose of an active ingredient of the pharmaceutical composition is 70 mg.
(102-w) The pharmaceutical composition according to (101) or (102), wherein a daily dose of an active ingredient of the pharmaceutical composition is 50 mg.
(102-x) The pharmaceutical composition according to (101) or (102), wherein a daily dose of an active ingredient of the pharmaceutical composition is 30 mg.
(102-y) The pharmaceutical composition according to (101) or (102), wherein a daily dose of an active ingredient of the pharmaceutical composition is 20 mg.
(102-z) The pharmaceutical composition according to (101) or (102), wherein a daily dose of an active ingredient of the pharmaceutical composition is 10 mg.

(103) The pharmaceutical composition according to any one of (101), (102), and (102-a) to (102-z), to be administered once daily.

(103-a) The pharmaceutical composition according to any one of (101), (102), (102-a) to (102-z), and (103), to be administered once daily after a meal.
(103-b) The pharmaceutical composition according to any one of (101), (102), (102-a) to (102-z), and (103), to be administered once daily at bedtime.
(103-c) The pharmaceutical composition according to any one of (101), (102), (102-a) to (102-z), and (103), to be administered once daily before a meal.
(103-d) The pharmaceutical composition according to any one of (101), (102), (102-a) to (102-z), and (103), to be administered once daily between meals.

(104) A method for treating, alleviating, and/or preventing interstitial cystitis and/or bladder pain syndrome, the method comprising the step of administering an effective amount of a compound represented by Formula (I):

[Chemical Formula 7]

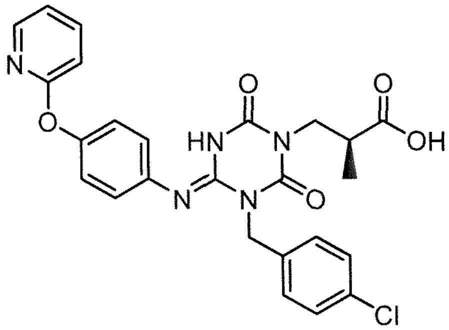

(I)

or a pharmaceutically acceptable salt thereof to an individual in need of treatment, alleviation, and/or prevention of interstitial cystitis and/or bladder pain syndrome.
(105) The treating, alleviating, and/or preventing method according to (104), wherein a daily dose of an active ingredient is 10 mg to 450 mg.

(105-a) The treating, alleviating, and/or preventing method according to (104) or (105), wherein a daily dose of an active ingredient is 10 mg to 300 mg.
(105-b) The treating, alleviating, and/or preventing method according to (104) or (105), wherein a daily dose of an active ingredient is 20 mg to 300 mg.
(105-c) The treating, alleviating, and/or preventing method according to (104) or (105), wherein a daily dose of an active ingredient is 30 mg to 300 mg.
(105-d) The treating, alleviating, and/or preventing method according to (104) or (105), wherein a daily dose of an active ingredient is 50 mg to 300 mg.
(105-e) The treating, alleviating, and/or preventing method according to (104) or (105), wherein a daily dose of an active ingredient is 70 mg to 300 mg.
(105-f) The treating, alleviating, and/or preventing method according to (104) or (105), wherein a daily dose of an active ingredient is 100 mg to 300 mg.
(105-g) The treating, alleviating, and/or preventing method according to (104) or (105), wherein a daily dose of an active ingredient is 150 mg to 300 mg.
(105-h) The treating, alleviating, and/or preventing method according to (104) or (105), wherein a daily dose of an active ingredient is 200 mg to 300 mg.
(105-i) The treating, alleviating, and/or preventing method according to (104) or (105), wherein a daily dose of an active ingredient is 10 mg to 150 mg.
(105-j) The treating, alleviating, and/or preventing method according to (104) or (105), wherein a daily dose of an active ingredient is 20 mg to 150 mg.
(105-k) The treating, alleviating, and/or preventing method according to (104) or (105), wherein a daily dose of an active ingredient is 30 mg to 150 mg.
(105-l) The treating, alleviating, and/or preventing method according to (104) or (105), wherein a daily dose of an active ingredient is 50 mg to 150 mg.
(105-m) The treating, alleviating, and/or preventing method according to (104) or (105), wherein a daily dose of an active ingredient is 70 mg to 150 mg.
(105-n) The treating, alleviating, and/or preventing method according to (104) or (105), wherein a daily dose of an active ingredient is 100 mg to 150 mg.

(105-o) The treating, alleviating, and/or preventing method according to (104) or (105), wherein a daily dose of an active ingredient is 50 mg to 200 mg.
(105-p) The treating, alleviating, and/or preventing method according to (104) or (105), wherein a daily dose of an active ingredient is 100 mg to 200 mg.
(105-q) The treating, alleviating, and/or preventing method according to (104) or (105), wherein a daily dose of an active ingredient is 150 mg to 200 mg.
(105-r) The treating, alleviating, and/or preventing method according to (104) or (105), wherein a daily dose of an active ingredient is 300 mg.
(105-s) The treating, alleviating, and/or preventing method according to (104) or (105), wherein a daily dose of an active ingredient is 200 mg.
(105-t) The treating, alleviating, and/or preventing method according to (104) or (105), wherein a daily dose of an active ingredient is 150 mg.
(105-u) The treating, alleviating, and/or preventing method according to (104) or (105), wherein a daily dose of an active ingredient is 100 mg.
(105-v) The treating, alleviating, and/or preventing method according to (104) or (105), wherein a daily dose of an active ingredient is 70 mg.
(105-w) The treating, alleviating, and/or preventing method according to (104) or (105), wherein a daily dose of an active ingredient is 50 mg.
(105-x) The treating, alleviating, and/or preventing method according to (104) or (105), wherein a daily dose of an active ingredient is 30 mg.
(105-y) The treating, alleviating, and/or preventing method according to (104) or (105), wherein a daily dose of an active ingredient is 20 mg.
(105-z) The treating, alleviating, and/or preventing method according to (104) or (105), wherein a daily dose of an active ingredient is 10 mg.

(106) The treating, alleviating, and/or preventing method according to any one of (104), (105), and (105-a) to (105-z), wherein the administering step is performed once daily.

(106-a) The treating, alleviating, and/or preventing method according to any one of (104), (105), (105-a) to (105-z), and (106), wherein the administering step is performed once daily after a meal.
(106-b) The treating, alleviating, and/or preventing method according to any one of (104), (105), (105-a) to (105-z), and (106), wherein the administering step is performed once daily at bedtime.
(106-c) The treating, alleviating, and/or preventing method according to any one of (104), (105), (105-a) to (105-z), and (106), wherein the administering step is performed once daily before a meal.
(106-d) The treating, alleviating, and/or preventing method according to any one of (104), (105), (105-a) to (105-z), and (106), wherein the administering step is performed once daily between meals.

(107) Use of a compound represented by Formula (I):

[Chemical Formula 8]

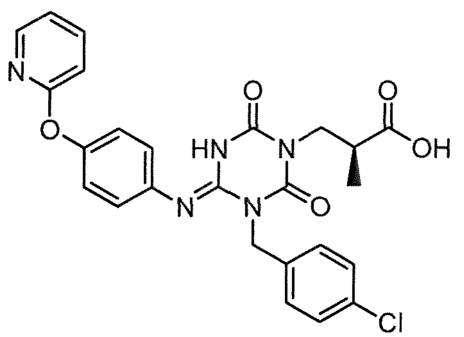

(I)

or a pharmaceutically acceptable salt thereof for manufacture of a medicament for treating, alleviating, and/or preventing interstitial cystitis and/or bladder pain syndrome.
(108) The use according to (107), wherein a daily dose of an active ingredient is 10 mg to 450 mg.

(108-a) The use according to (107) or (108), wherein a daily dose of an active ingredient is 10 mg to 300 mg.
(108-b) The use according to (107) or (108), wherein a daily dose of an active ingredient is 20 mg to 300 mg.
(108-c) The use according to (107) or (108), wherein a daily dose of an active ingredient is 30 mg to 300 mg.
(108-d) The use according to (107) or (108), wherein a daily dose of an active ingredient is 50 mg to 300 mg.
(108-e) The use according to (107) or (108), wherein a daily dose of an active ingredient is 70 mg to 300 mg.
(108-f) The use according to (107) or (108), wherein a daily dose of an active ingredient is 100 mg to 300 mg.
(108-g) The use according to (107) or (108), wherein a daily dose of an active ingredient is 150 mg to 300 mg.
(108-h) The use according to (107) or (108), wherein a daily dose of an active ingredient is 200 mg to 300 mg.
(108-i) The use according to (107) or (108), wherein a daily dose of an active ingredient is 10 mg to 150 mg.
(108-j) The use according to (107) or (108), wherein a daily dose of an active ingredient is 20 mg to 150 mg.
(108-k) The use according to (107) or (108), wherein a daily dose of an active ingredient is 30 mg to 150 mg.
(108-l) The use according to (107) or (108), wherein a daily dose of an active ingredient is 50 mg to 150 mg.
(108-m) The use according to (107) or (108), wherein a daily dose of an active ingredient is 70 mg to 150 mg.
(108-n) The use according to (107) or (108), wherein a daily dose of an active ingredient is 100 mg to 150 mg.
(108-o) The use according to (107) or (108), wherein a daily dose of an active ingredient is 50 mg to 200 mg.
(108-p) The use according to (107) or (108), wherein a daily dose of an active ingredient is 100 mg to 200 mg.
(108-q) The use according to (107) or (108), wherein a daily dose of an active ingredient is 150 mg to 200 mg.
(108-r) The use according to (107) or (108), wherein a daily dose of an active ingredient is 300 mg.
(108-s) The use according to (107) or (108), wherein a daily dose of an active ingredient is 200 mg.
(108-t) The use according to (107) or (108), wherein a daily dose of an active ingredient is 150 mg.
(108-u) The use according to (107) or (108), wherein a daily dose of an active ingredient is 100 mg.
(108-v) The use according to (107) or (108), wherein a daily dose of an active ingredient is 70 mg.
(108-w) The use according to (107) or (108), wherein a daily dose of an active ingredient is 50 mg.
(108-x) The use according to (107) or (108), wherein a daily dose of an active ingredient is 30 mg.
(108-y) The use according to (107) or (108), wherein a daily dose of an active ingredient is 20 mg.
(108-z) The use according to (107) or (108), wherein a daily dose of an active ingredient is 10 mg.

(109) The use according to any one of (107), (108), and (108-a) to (108-z), wherein the medicament is to be administered once daily.

(109-a) The use according to any one of (107), (108), (108-a) to (108-z), and (109), wherein the medicament is to be administered once daily after a meal.
(109-b) The use according to any one of (107), (108), (108-a) to (108-z), and (109), wherein the medicament is to be administered once daily at bedtime.
(109-c) The use according to any one of (107), (108), (108-a) to (108-z), and (109), wherein the medicament is to be administered once daily before a meal.
(109-d) The use according to any one of (107), (108), (108-a) to (108-z), and (109), wherein the medicament is to be administered once daily between meals.

(110) A compound represented by Formula (I):

[Chemical Formula 9]

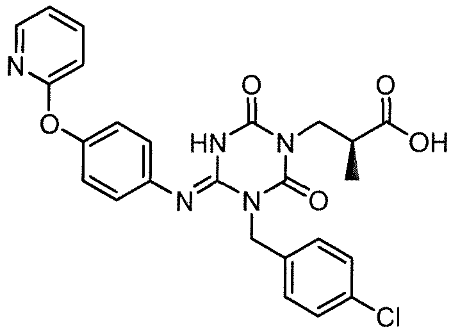

(I)

or a pharmaceutically acceptable salt thereof to be used for treating, alleviating, and/or preventing interstitial cystitis and/or bladder pain syndrome.
(111) The compound according to (110) or a pharmaceutically acceptable salt thereof, wherein a daily dose of an active ingredient is 10 mg to 450 mg.

(111-a) The compound according to (110) or (111) or a pharmaceutically acceptable salt thereof, wherein a daily dose of an active ingredient is 10 mg to 300 mg.
(111-b) The compound according to (110) or (111) or a pharmaceutically acceptable salt thereof, wherein a daily dose of an active ingredient is 20 mg to 300 mg.
(111-c) The compound according to (110) or (111) or a pharmaceutically acceptable salt thereof, wherein a daily dose of an active ingredient is 30 mg to 300 mg.
(111-d) The compound according to (110) or (111) or a pharmaceutically acceptable salt thereof, wherein a daily dose of an active ingredient is 50 mg to 300 mg.
(111-e) The compound according to (110) or (111) or a pharmaceutically acceptable salt thereof, wherein a daily dose of an active ingredient is 70 mg to 300 mg.
(111-f) The compound according to (110) or (111) or a pharmaceutically acceptable salt thereof, wherein a daily dose of an active ingredient is 100 mg to 300 mg.
(111-g) The compound according to (110) or (111) or a pharmaceutically acceptable salt thereof, wherein a daily dose of an active ingredient is 150 mg to 300 mg.
(111 -h) The compound according to (110) or (111) or a pharmaceutically acceptable salt thereof, wherein a daily dose of an active ingredient is 200 mg to 300 mg.
(111-i) The compound according to (110) or (111) or a pharmaceutically acceptable salt thereof, wherein a daily dose of an active ingredient is 10 mg to 150 mg.
(111-j) The compound according to (110) or (111) or a pharmaceutically acceptable salt thereof, wherein a daily dose of an active ingredient is 20 mg to 150 mg.
(111-k) The compound according to (110) or (111) or a pharmaceutically acceptable salt thereof, wherein a daily dose of an active ingredient is 30 mg to 150 mg.
(111-l) The compound according to (110) or (111) or a pharmaceutically acceptable salt thereof, wherein a daily dose of an active ingredient is 50 mg to 150 mg.
(111-m) The compound according to (110) or (111) or a pharmaceutically acceptable salt thereof, wherein a daily dose of an active ingredient is 70 mg to 150 mg.
(111-n) The compound according to (110) or (111) or a pharmaceutically acceptable salt thereof, wherein a daily dose of an active ingredient is 100 mg to 150 mg.
(111-o) The compound according to (110) or (111) or a pharmaceutically acceptable salt thereof, wherein a daily dose of an active ingredient is 50 mg to 200 mg.
(111-p) The compound according to (110) or (111) or a pharmaceutically acceptable salt thereof, wherein a daily dose of an active ingredient is 100 mg to 200 mg.
(111-q) The compound according to (110) or (111) or a pharmaceutically acceptable salt thereof, wherein a daily dose of an active ingredient is 150 mg to 200 mg.
(111-r) The compound according to (110) or (111) or a pharmaceutically acceptable salt thereof, wherein a daily dose of an active ingredient is 300 mg.
(111-s) The compound according to (110) or (111) or a pharmaceutically acceptable salt thereof, wherein a daily dose of an active ingredient is 200 mg.
(111-t) The compound according to (110) or (111) or a pharmaceutically acceptable salt thereof, wherein a daily dose of an active ingredient is 150 mg.
(111-u) The compound according to (110) or (111) or a pharmaceutically acceptable salt thereof, wherein a daily dose of an active ingredient is 100 mg.
(111-v) The compound according to (110) or (111) or a pharmaceutically acceptable salt thereof, wherein a daily dose of an active ingredient is 70 mg.
(111-w) The compound according to (110) or (111) or a pharmaceutically acceptable salt thereof, wherein a daily dose of an active ingredient is 50 mg.
(111-x) The compound according to (110) or (111) or a pharmaceutically acceptable salt thereof, wherein a daily dose of an active ingredient is 30 mg.
(111-y) The compound according to (110) or (111) or a pharmaceutically acceptable salt thereof, wherein a daily dose of an active ingredient is 20 mg.
(111-z) The compound according to (110) or (111) or a pharmaceutically acceptable salt thereof, wherein a daily dose of an active ingredient is 10 mg.

(112) The compound according to any one of (110), (111), and (111-a) to (111-z) or a pharmaceutically acceptable salt thereof, to be administered once daily.
(112-a) The compound according to any one of (110), (111), (111-a) to (111-z), and (112) or a pharmaceutically acceptable salt thereof, to be administered once daily after a meal.
(112-b) The compound according to any one of (110), (111), (111-a) to (111-z), and (112) or a pharmaceutically

acceptable salt thereof, to be administered once daily at bedtime.

(112-c) The compound according to any one of (110), (111), (111-a) to (111-z), and (112) or a pharmaceutically acceptable salt thereof, to be administered once daily before a meal.

(112-d) The compound according to any one of (110), (111), (111-a) to (111-z), and (112) or a pharmaceutically acceptable salt thereof, to be administered once daily between meals.

Neuropathic pain encompasses peripheral neuropathic pain, diabetic neuropathic pain, postoperative neuropathic pain, postherpetic neuralgia, trigeminal neuralgia, neuropathic low back pain, and cancer pain.

Neuropathic pain encompasses postherpetic neuralgia, trigeminal neuralgia, neuropathic low back pain, sciatica, neuropathic pain associated with cervical spondylosis, pain associated with lumbar spinal canal stenosis, pain associated with cervical radiculopathy, phantom limb pain, and complex regional pain syndrome.

Examples of the site of onset of neuropathic pain include the waist, the foot, the neck, the shoulder, the arm, the buttocks, and the face. Neuropathic pain is pain caused by damage or dysfunction of the nerve, spinal cord, or brain, and examples of the cause include compression of the nerve (examples of which include a tumor, disc rupture, or compression of nerves in the wrist (causing carpal tunnel syndrome).

Other causes include nerve damage, including diseases that affect the whole body (such as diabetes) and diseases that affect parts of the body (such as herpes zoster).

Furthermore, neuropathic pain encompasses pain that occurs after breast removal (mastectomy) or lung surgery (thoracotomy).

When a lesion or disease is present in any of the nociceptive information transmission pathways from the peripheral nerve to the cerebrum, hypersensitivity of a nerve response appears in the nervous system of greater than or equal to the dorsal horn nerve cells even without excitement of the nociceptor on the peripheral nerve terminal, and hyperalgesia, allodynia, and spontaneous pain appear. Such hypersensitivity of a nerve response is considered to be neuropathic pain, and a pharmaceutical composition comprising the compound represented by Formula (I) or a pharmaceutically acceptable salt thereof exhibits efficacy against the pain described above.

Nociceptive pain encompasses inflammatory pain, knee osteoarthritis pain, joint pain associated with rheumatoid arthritis, and cancer pain.

Visceral pain encompasses cystitis pain, cancer pain, and pain associated with endometriosis.

Cystitis pain encompasses pain associated with interstitial cystitis, pain associated with uncomplicated cystitis, pain associated with complicated cystitis, pain associated with cystic cystitis, and pain associated with fungal cystitis.

Cystitis pain encompasses pain associated with urination (urination pain).

A pharmaceutical composition comprising a compound represented by Formula (I) or a pharmaceutically acceptable salt thereof is effective in treating, alleviating, and/or preventing bladder pain syndrome.

Bladder pain syndrome means an unpleasant sensation associated with the bladder (pain, pressure, discomfort) that involves lower urinary tract symptoms and lacks an infection or other probable cause.

A pharmaceutical composition for treating, alleviating, and/or preventing neuropathic pain, nociceptive pain, or visceral pain, containing 10 mg of a compound represented by Formula (I) or a pharmaceutically acceptable salt thereof, is included as an embodiment.

A method for treating, alleviating, and/or preventing neuropathic pain, nociceptive pain, or visceral pain, the method including administering 10 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing neuropathic pain, nociceptive pain, or visceral pain, the medicament containing 10 mg of the compound represented by Formula (I) or the pharmaceutically acceptable salt thereof, is included as an embodiment.

A pharmaceutical composition for treating, alleviating, and/or preventing peripheral neuropathic pain, containing 10 mg of a compound represented by Formula (I) or a pharmaceutically acceptable salt thereof, is included as an embodiment.

A method for treating, alleviating, and/or preventing peripheral neuropathic pain, the method including administering 10 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing peripheral neuropathic pain, the medicament containing 10 mg of the compound represented by Formula (I) or the pharmaceutically acceptable salt thereof, is included as an embodiment.

A pharmaceutical composition for treating, alleviating, and/or preventing diabetic neuropathic pain, containing 10 mg of a compound represented by Formula (I) or a pharmaceutically acceptable salt thereof, is included as an embodiment.

A method for treating, alleviating, and/or preventing diabetic neuropathic pain, the method including administering 10 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing diabetic neuropathic pain, the medicament containing 10 mg of the compound represented by Formula (I) or the pharmaceutically acceptable salt thereof, is included as an embodiment.

A pharmaceutical composition for treating, alleviating, and/or preventing cystitis pain, containing 10 mg of a compound represented by Formula (I) or a pharmaceutically acceptable salt thereof, is included as an embodiment.

A method for treating, alleviating, and/or preventing cystitis pain, the method including administering 10 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing cystitis pain, the medicament containing 10 mg of the compound represented by Formula (I) or the pharmaceutically acceptable salt thereof, is included as an embodiment.

A pharmaceutical composition for treating, alleviating, and/or preventing pain associated with endometriosis, containing 10 mg of a compound represented by Formula (I) or a pharmaceutically acceptable salt thereof, is included as an embodiment.

A method for treating, alleviating, and/or preventing pain associated with endometriosis, the method including administering 10 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing pain associated with endometriosis, the medicament containing 10 mg of the compound represented by Formula (I) or the pharmaceutically acceptable salt thereof, is included as an embodiment.

A pharmaceutical composition for treating, alleviating, and/or preventing interstitial cystitis and/or bladder pain syndrome, containing 10 mg of a compound represented by Formula (I) or a pharmaceutically acceptable salt thereof, is included as an embodiment.

A method for treating, alleviating, and/or preventing interstitial cystitis and/or bladder pain syndrome, the method including administering 10 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing interstitial cystitis and/or bladder pain syndrome, the medicament containing 10 mg of the compound represented by Formula (I) or the pharmaceutically acceptable salt thereof, is included as an embodiment.

A pharmaceutical composition for treating, alleviating, and/or preventing neuropathic pain, nociceptive pain, or visceral pain, containing 20 mg of a compound represented by Formula (I) or a pharmaceutically acceptable salt thereof, is included as an embodiment.

A method for treating, alleviating, and/or preventing neuropathic pain, nociceptive pain, or visceral pain, the method including administering 20 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing neuropathic pain, nociceptive pain, or visceral pain, the medicament containing 20 mg of the compound represented by Formula (I) or the pharmaceutically acceptable salt thereof, is included as an embodiment.

A pharmaceutical composition for treating, alleviating, and/or preventing peripheral neuropathic pain, containing 20 mg of a compound represented by Formula (I) or a pharmaceutically acceptable salt thereof, is included as an embodiment.

A method for treating, alleviating, and/or preventing peripheral neuropathic pain, the method including administering 20 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing peripheral neuropathic pain, the medicament containing 20 mg of the compound represented by Formula (I) or the pharmaceutically acceptable salt thereof, is included as an embodiment.

A pharmaceutical composition for treating, alleviating, and/or preventing diabetic neuropathic pain, containing 20 mg of a compound represented by Formula (I) or a pharmaceutically acceptable salt thereof, is included as an embodiment.

A method for treating, alleviating, and/or preventing diabetic neuropathic pain, the method including administering 20 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing diabetic neuropathic pain, the medicament containing 20 mg of the compound represented by Formula (I) or the pharmaceutically acceptable salt thereof, is included as an embodiment.

A pharmaceutical composition for treating, alleviating, and/or preventing cystitis pain, containing 20 mg of a compound represented by Formula (I) or a pharmaceutically acceptable salt thereof, is included as an embodiment.

A method for treating, alleviating, and/or preventing cystitis pain, the method including administering 20 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing cystitis pain, the medicament containing 20 mg of the compound represented by Formula (I) or the pharmaceutically acceptable salt thereof, is included as an embodiment.

A pharmaceutical composition for treating, alleviating, and/or preventing pain associated with endometriosis, containing 20 mg of a compound represented by Formula (I) or a pharmaceutically acceptable salt thereof, is included as an embodiment.

A method for treating, alleviating, and/or preventing pain associated with endometriosis, the method including administering 20 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing pain associated with endometriosis, the medicament containing 20 mg of the compound represented by Formula (I) or the pharmaceutically acceptable salt thereof, is included as an embodiment.

A pharmaceutical composition for treating, alleviating, and/or preventing interstitial cystitis and/or bladder pain syndrome, containing 20 mg of a compound represented by Formula (I) or a pharmaceutically acceptable salt thereof, is included as an embodiment.

A method for treating, alleviating, and/or preventing interstitial cystitis and/or bladder pain syndrome, the method including administering 20 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing interstitial cystitis and/or bladder pain syndrome, the medicament containing 20 mg of the compound represented by Formula (I) or the pharmaceutically acceptable salt thereof, is included as an embodiment.

A pharmaceutical composition for treating, alleviating, and/or preventing neuropathic pain, nociceptive pain, or visceral pain, containing 30 mg of a compound represented by Formula (I) or a pharmaceutically acceptable salt thereof, is included as an embodiment.

A method for treating, alleviating, and/or preventing neuropathic pain, nociceptive pain, or visceral pain, the method including administering 30 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing neuropathic pain, nociceptive pain, or visceral pain, the medicament containing 30 mg of the compound represented by Formula (I) or the pharmaceutically acceptable salt thereof, is included as an embodiment.

A pharmaceutical composition for treating, alleviating, and/or preventing peripheral neuropathic pain, containing 30 mg of a compound represented by Formula (I) or a pharmaceutically acceptable salt thereof, is included as an embodiment.

A method for treating, alleviating, and/or preventing peripheral neuropathic pain, the method including administering 30 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing peripheral neuropathic pain, the medicament containing 30 mg of the compound represented by Formula (I) or the pharmaceutically acceptable salt thereof, is included as an embodiment.

A pharmaceutical composition for treating, alleviating, and/or preventing diabetic neuropathic pain, containing 30 mg of a compound represented by Formula (I) or a pharmaceutically acceptable salt thereof, is included as an embodiment.

A method for treating, alleviating, and/or preventing diabetic neuropathic pain, the method including administering 30 mg of a compound represented by Formula (I) or a pharmaceutically acceptable salt thereof, is included as an

embodiment.

Use of a compound represented by Formula (I) or a pharmaceutically acceptable salt thereof for manufacture of a medicament for treating, alleviating, and/or preventing diabetic neuropathic pain, the medicament containing 30 mg of the compound represented by Formula (I) or the pharmaceutically acceptable salt thereof, is included as an embodiment.

A pharmaceutical composition for treating, alleviating, and/or preventing cystitis pain, containing 30 mg of a compound represented by Formula (I) or a pharmaceutically acceptable salt thereof, is included as an embodiment.

A method for treating, alleviating, and/or preventing cystitis pain, the method including administering 30 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing cystitis pain, the medicament containing 30 mg of the compound represented by Formula (I) or the pharmaceutically acceptable salt thereof, is included as an embodiment.

A pharmaceutical composition for treating, alleviating, and/or preventing pain associated with endometriosis, containing 30 mg of a compound represented by Formula (I) or a pharmaceutically acceptable salt thereof, is included as an embodiment.

A method for treating, alleviating, and/or preventing pain associated with endometriosis, the method including administering 30 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing pain associated with endometriosis, the medicament containing 30 mg of the compound represented by Formula (I) or the pharmaceutically acceptable salt thereof, is included as an embodiment.

A pharmaceutical composition for treating, alleviating, and/or preventing interstitial cystitis and/or bladder pain syndrome, containing 30 mg of a compound represented by Formula (I) or a pharmaceutically acceptable salt thereof, is included as an embodiment.

A method for treating, alleviating, and/or preventing interstitial cystitis and/or bladder pain syndrome, the method including administering 30 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing interstitial cystitis and/or bladder pain syndrome, the medicament containing 30 mg of the compound represented by Formula (I) or the pharmaceutically acceptable salt thereof, is included as an embodiment.

A pharmaceutical composition for treating, alleviating, and/or preventing neuropathic pain, nociceptive pain, or visceral pain, containing 50 mg of a compound represented by Formula (I) or a pharmaceutically acceptable salt thereof, is included as an embodiment.

A method for treating, alleviating, and/or preventing neuropathic pain, nociceptive pain, or visceral pain, the method including administering 50 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing neuropathic pain, nociceptive pain, or visceral pain, the medicament containing 50 mg of the compound represented by Formula (I) or the pharmaceutically acceptable salt thereof, is included as an embodiment.

A pharmaceutical composition for treating, alleviating, and/or preventing peripheral neuropathic pain, containing 50 mg of a compound represented by Formula (I) or a pharmaceutically acceptable salt thereof, is included as an embodiment.

A method for treating, alleviating, and/or preventing peripheral neuropathic pain, the method including administering 50 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing peripheral neuropathic pain, the medicament containing 50 mg of the compound represented by Formula (I) or the pharmaceutically acceptable salt thereof, is included as an embodiment.

A pharmaceutical composition for treating, alleviating, and/or preventing diabetic neuropathic pain, containing 50 mg of a compound represented by Formula (I) or a pharmaceutically acceptable salt thereof, is included as an embodiment.

A method for treating, alleviating, and/or preventing diabetic neuropathic pain, the method including administering 50 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing diabetic neuropathic pain, the medicament containing 50 mg of the compound represented by Formula (I) or the pharmaceutically acceptable salt thereof, is included as an embodiment.

A pharmaceutical composition for treating, alleviating, and/or preventing cystitis pain, containing 50 mg of a compound represented by Formula (I) or a pharmaceutically acceptable salt thereof, is included as an embodiment.

A method for treating, alleviating, and/or preventing cystitis pain, the method including administering 50 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing cystitis pain, the medicament containing 50 mg of the compound represented by Formula (I) or the pharmaceutically acceptable salt thereof, is included as an embodiment.

A pharmaceutical composition for treating, alleviating, and/or preventing pain associated with endometriosis, containing 50 mg of a compound represented by Formula (I) or a pharmaceutically acceptable salt thereof, is included as an embodiment.

A method for treating, alleviating, and/or preventing pain associated with endometriosis, the method including administering 50 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing pain associated with endometriosis, the medicament containing 50 mg of the compound represented by Formula (I) or the pharmaceutically acceptable salt thereof, is included as an embodiment.

A pharmaceutical composition for treating, alleviating, and/or preventing interstitial cystitis and/or bladder pain syndrome, containing 50 mg of a compound represented by Formula (I) or a pharmaceutically acceptable salt thereof, is included as an embodiment.

A method for treating, alleviating, and/or preventing interstitial cystitis and/or bladder pain syndrome, the method including administering 50 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing interstitial cystitis and/or bladder pain syndrome, the medicament containing 50 mg of the compound represented by Formula (I) or the pharmaceutically acceptable salt thereof, is included as an embodiment.

A pharmaceutical composition for treating, alleviating, and/or preventing neuropathic pain, nociceptive pain, or visceral pain, containing 70 mg of a compound represented by Formula (I) or a pharmaceutically acceptable salt thereof, is included as an embodiment.

A method for treating, alleviating, and/or preventing neuropathic pain, nociceptive pain, or visceral pain, the method including administering 70 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing neuropathic pain, nociceptive pain, or visceral pain, the medicament containing 70 mg of the compound represented by Formula (I) or the pharmaceutically acceptable salt thereof, is included as an embodiment.

A pharmaceutical composition for treating, alleviating, and/or preventing peripheral neuropathic pain, containing 70 mg of a compound represented by Formula (I) or a pharmaceutically acceptable salt thereof, is included as an embodiment.

A method for treating, alleviating, and/or preventing peripheral neuropathic pain, the method including administering 70 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing peripheral neuropathic pain, the medicament containing 70 mg of the compound represented by Formula (I) or the pharmaceutically acceptable salt thereof, is included as an embodiment.

A pharmaceutical composition for treating, alleviating, and/or preventing diabetic neuropathic pain, containing 70 mg of a compound represented by Formula (I) or a pharmaceutically acceptable salt thereof, is included as an embodiment.

A method for treating, alleviating, and/or preventing diabetic neuropathic pain, the method including administering 70 mg of a compound represented by Formula (I) or a pharmaceutically acceptable salt thereof, is included as an embodiment.

Use of a compound represented by Formula (I) or a pharmaceutically acceptable salt thereof for manufacture

of a medicament for treating, alleviating, and/or preventing diabetic neuropathic pain, the medicament containing 70 mg of the compound represented by Formula (I) or the pharmaceutically acceptable salt thereof, is included as an embodiment.

A pharmaceutical composition for treating, alleviating, and/or preventing cystitis pain, containing 70 mg of a compound represented by Formula (I) or a pharmaceutically acceptable salt thereof, is included as an embodiment.

A method for treating, alleviating, and/or preventing cystitis pain, the method including administering 70 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing cystitis pain, the medicament containing 70 mg of the compound represented by Formula (I) or the pharmaceutically acceptable salt thereof, is included as an embodiment.

A pharmaceutical composition for treating, alleviating, and/or preventing pain associated with endometriosis, containing 70 mg of a compound represented by Formula (I) or a pharmaceutically acceptable salt thereof, is included as an embodiment.

A method for treating, alleviating, and/or preventing pain associated with endometriosis, the method including administering 70 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing pain associated with endometriosis, the medicament containing 70 mg of the compound represented by Formula (I) or the pharmaceutically acceptable salt thereof, is included as an embodiment.

A pharmaceutical composition for treating, alleviating, and/or preventing interstitial cystitis and/or bladder pain syndrome, containing 70 mg of a compound represented by Formula (I) or a pharmaceutically acceptable salt thereof, is included as an embodiment.

A method for treating, alleviating, and/or preventing interstitial cystitis and/or bladder pain syndrome, the method including administering 70 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing interstitial cystitis and/or bladder pain syndrome, the medicament containing 70 mg of the compound represented by Formula (I) or the pharmaceutically acceptable salt thereof, is included as an embodiment.

A pharmaceutical composition for treating, alleviating, and/or preventing neuropathic pain, nociceptive pain, or visceral pain, containing 100 mg of a compound represented by Formula (I) or a pharmaceutically acceptable salt thereof, is included as an embodiment.

A method for treating, alleviating, and/or preventing neuropathic pain, nociceptive pain, or visceral pain, the method including administering 100 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing neuropathic pain, nociceptive pain, or visceral pain, the medicament containing 100 mg of the compound represented by Formula (I) or the pharmaceutically acceptable salt thereof, is included as an embodiment.

A pharmaceutical composition for treating, alleviating, and/or preventing peripheral neuropathic pain, containing 100 mg of a compound represented by Formula (I) or a pharmaceutically acceptable salt thereof, is included as an embodiment.

A method for treating, alleviating, and/or preventing peripheral neuropathic pain, the method including administering 100 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing peripheral neuropathic pain, the medicament containing 100 mg of the compound represented by Formula (I) or the pharmaceutically acceptable salt thereof, is included as an embodiment.

A pharmaceutical composition for treating, alleviating, and/or preventing diabetic neuropathic pain, containing 100 mg of a compound represented by Formula (1) or a pharmaceutically acceptable salt thereof, is included as an embodiment.

A method for treating, alleviating, and/or preventing diabetic neuropathic pain, the method including administering 100 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing diabetic neuropathic pain, the medicament containing 100

mg of the compound represented by Formula (I) or the pharmaceutically acceptable salt thereof, is included as an embodiment.

A pharmaceutical composition for treating, alleviating, and/or preventing cystitis pain, containing 100 mg of a compound represented by Formula (I) or a pharmaceutically acceptable salt thereof, is included as an embodiment.

A method for treating, alleviating, and/or preventing cystitis pain, the method including administering 100 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing cystitis pain, the medicament containing 100 mg of the compound represented by Formula (I) or the pharmaceutically acceptable salt thereof, is included as an embodiment.

A pharmaceutical composition for treating, alleviating, and/or preventing pain associated with endometriosis, containing 100 mg of a compound represented by Formula (I) or a pharmaceutically acceptable salt thereof, is included as an embodiment.

A method for treating, alleviating, and/or preventing pain associated with endometriosis, the method including administering 100 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing pain associated with endometriosis, the medicament containing 100 mg of the compound represented by Formula (I) or the pharmaceutically acceptable salt thereof, is included as an embodiment.

A pharmaceutical composition for treating, alleviating, and/or preventing interstitial cystitis and/or bladder pain syndrome, containing 100 mg of a compound represented by Formula (I) or a pharmaceutically acceptable salt thereof, is included as an embodiment.

A method for treating, alleviating, and/or preventing interstitial cystitis and/or bladder pain syndrome, the method including administering 100 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing interstitial cystitis and/or bladder pain syndrome, the medicament containing 100 mg of the compound represented by Formula (I) or the pharmaceutically acceptable salt thereof, is included as an embodiment.

A pharmaceutical composition for treating, alleviating, and/or preventing neuropathic pain, nociceptive pain, or visceral pain, containing 150 mg of a compound represented by Formula (I) or a pharmaceutically acceptable salt thereof, is included as an embodiment.

A method for treating, alleviating, and/or preventing neuropathic pain, nociceptive pain, or visceral pain, the method including administering 150 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing neuropathic pain, nociceptive pain, or visceral pain, the medicament containing 150 mg of the compound represented by Formula (I) or the pharmaceutically acceptable salt thereof, is included as an embodiment.

A pharmaceutical composition for treating, alleviating, and/or preventing peripheral neuropathic pain, containing 150 mg of a compound represented by Formula (I) or a pharmaceutically acceptable salt thereof, is included as an embodiment.

A method for treating, alleviating, and/or preventing peripheral neuropathic pain, the method including administering 150 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing peripheral neuropathic pain, the medicament containing 150 mg of the compound represented by Formula (I) or the pharmaceutically acceptable salt thereof, is included as an embodiment.

A pharmaceutical composition for treating, alleviating, and/or preventing diabetic neuropathic pain, containing 150 mg of a compound represented by Formula (I) or a pharmaceutically acceptable salt thereof, is included as an embodiment.

A method for treating, alleviating, and/or preventing diabetic neuropathic pain, the method including administering 150 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing diabetic neuropathic pain, the medicament containing 150 mg of the compound represented by Formula (I) or the pharmaceutically acceptable salt thereof, is included as an

embodiment.

A pharmaceutical composition for treating, alleviating, and/or preventing cystitis pain, containing 150 mg of a compound represented by Formula (I) or a pharmaceutically acceptable salt thereof, is included as an embodiment.

A method for treating, alleviating, and/or preventing cystitis pain, the method including administering 150 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing cystitis pain, the medicament containing 150 mg of the compound represented by Formula (I) or the pharmaceutically acceptable salt thereof, is included as an embodiment.

A pharmaceutical composition for treating, alleviating, and/or preventing pain associated with endometriosis, containing 150 mg of a compound represented by Formula (I) or a pharmaceutically acceptable salt thereof, is included as an embodiment.

A method for treating, alleviating, and/or preventing pain associated with endometriosis, the method including administering 150 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing pain associated with endometriosis, the medicament containing 150 mg of the compound represented by Formula (I) or the pharmaceutically acceptable salt thereof, is included as an embodiment.

A pharmaceutical composition for treating, alleviating, and/or preventing interstitial cystitis and/or bladder pain syndrome, containing 150 mg of a compound represented by Formula (I) or a pharmaceutically acceptable salt thereof, is included as an embodiment.

A method for treating, alleviating, and/or preventing interstitial cystitis and/or bladder pain syndrome, the method including administering 150 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing interstitial cystitis and/or bladder pain syndrome, the medicament containing 150 mg of the compound represented by Formula (I) or the pharmaceutically acceptable salt thereof, is included as an embodiment.

A pharmaceutical composition for treating, alleviating, and/or preventing neuropathic pain, nociceptive pain, or visceral pain, containing 200 mg of a compound represented by Formula (I) or a pharmaceutically acceptable salt thereof, is included as an embodiment.

A method for treating, alleviating, and/or preventing neuropathic pain, nociceptive pain, or visceral pain, the method including administering 200 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing neuropathic pain, nociceptive pain, or visceral pain, the medicament containing 200 mg of the compound represented by Formula (I) or the pharmaceutically acceptable salt thereof, is included as an embodiment.

A pharmaceutical composition for treating, alleviating, and/or preventing peripheral neuropathic pain, containing 200 mg of a compound represented by Formula (I) or a pharmaceutically acceptable salt thereof, is included as an embodiment.

A method for treating, alleviating, and/or preventing peripheral neuropathic pain, the method including administering 200 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing peripheral neuropathic pain, the medicament containing 200 mg of the compound represented by Formula (I) or the pharmaceutically acceptable salt thereof, is included as an embodiment.

A pharmaceutical composition for treating, alleviating, and/or preventing diabetic neuropathic pain, containing 200 mg of a compound represented by Formula (I) or a pharmaceutically acceptable salt thereof, is included as an embodiment.

A method for treating, alleviating, and/or preventing diabetic neuropathic pain, the method including administering 200 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing diabetic neuropathic pain, the medicament containing 200 mg of the compound represented by Formula (I) or the pharmaceutically acceptable salt thereof, is included as an embodiment.

A pharmaceutical composition for treating, alleviating, and/or preventing cystitis pain, containing 200 mg of a compound represented by Formula (I) or a pharmaceutically acceptable salt thereof, is included as an embodiment.

A method for treating, alleviating, and/or preventing cystitis pain, the method including administering 200 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing cystitis pain, the medicament containing 200 mg of the compound represented by Formula (I) or the pharmaceutically acceptable salt thereof, is included as an embodiment.

A pharmaceutical composition for treating, alleviating, and/or preventing pain associated with endometriosis, containing 200 mg of a compound represented by Formula (I) or a pharmaceutically acceptable salt thereof, is included as an embodiment.

A method for treating, alleviating, and/or preventing pain associated with endometriosis, the method including administering 200 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing pain associated with endometriosis, the medicament containing 200 mg of the compound represented by Formula (I) or the pharmaceutically acceptable salt thereof, is included as an embodiment.

A pharmaceutical composition for treating, alleviating, and/or preventing interstitial cystitis and/or bladder pain syndrome, containing 200 mg of a compound represented by Formula (I) or a pharmaceutically acceptable salt thereof, is included as an embodiment.

A method for treating, alleviating, and/or preventing interstitial cystitis and/or bladder pain syndrome, the method including administering 200 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing interstitial cystitis and/or bladder pain syndrome, the medicament containing 200 mg of the compound represented by Formula (I) or the pharmaceutically acceptable salt thereof, is included as an embodiment.

A pharmaceutical composition for treating, alleviating, and/or preventing neuropathic pain, nociceptive pain, or visceral pain, containing 300 mg of a compound represented by Formula (I) or a pharmaceutically acceptable salt thereof, is included as an embodiment.

A method for treating, alleviating, and/or preventing neuropathic pain, nociceptive pain, or visceral pain, the method including administering 300 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing neuropathic pain, nociceptive pain, or visceral pain, the medicament containing 300 mg of the compound represented by Formula (I) or the pharmaceutically acceptable salt thereof, is included as an embodiment.

A pharmaceutical composition for treating, alleviating, and/or preventing peripheral neuropathic pain, containing 300 mg of a compound represented by Formula (I) or a pharmaceutically acceptable salt thereof, is included as an embodiment.

A method for treating, alleviating, and/or preventing peripheral neuropathic pain, the method including administering 300 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing peripheral neuropathic pain, the medicament containing 300 mg of the compound represented by Formula (I) or the pharmaceutically acceptable salt thereof, is included as an embodiment.

A pharmaceutical composition for treating, alleviating, and/or preventing diabetic neuropathic pain, containing 300 mg of a compound represented by Formula (I) or a pharmaceutically acceptable salt thereof, is included as an embodiment.

A method for treating, alleviating, and/or preventing diabetic neuropathic pain, the method including administering 300 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing diabetic neuropathic pain, the medicament containing 300 mg of the compound represented by Formula (I) or the pharmaceutically acceptable salt thereof, is included as an embodiment.

A pharmaceutical composition for treating, alleviating, and/or preventing cystitis pain, containing 300 mg of a

compound represented by Formula (I) or a pharmaceutically acceptable salt thereof, is included as an embodiment.

A method for treating, alleviating, and/or preventing cystitis pain, the method including administering 300 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing cystitis pain, the medicament containing 300 mg of the compound represented by Formula (I) or the pharmaceutically acceptable salt thereof, is included as an embodiment.

A pharmaceutical composition for treating, alleviating, and/or preventing pain associated with endometriosis, containing 300 mg of a compound represented by Formula (I) or a pharmaceutically acceptable salt thereof, is included as an embodiment.

A method for treating, alleviating, and/or preventing pain associated with endometriosis, the method including administering 300 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing pain associated with endometriosis, the medicament containing 300 mg of the compound represented by Formula (I) or the pharmaceutically acceptable salt thereof, is included as an embodiment.

A pharmaceutical composition for treating, alleviating, and/or preventing interstitial cystitis and/or bladder pain syndrome, containing 300 mg of a compound represented by Formula (I) or a pharmaceutically acceptable salt thereof, is included as an embodiment.

A method for treating, alleviating, and/or preventing interstitial cystitis and/or bladder pain syndrome, the method including administering 300 mg of a compound represented by Formula (I) or a pharmaceutically acceptable salt thereof, is included as an embodiment.

Use of a compound represented by Formula (I) or a pharmaceutically acceptable salt thereof for manufacture of a medicament for treating, alleviating, and/or preventing interstitial cystitis and/or bladder pain syndrome, the medicament containing 300 mg of the compound represented by Formula (I) or the pharmaceutically acceptable salt thereof, is included as an embodiment.

[EFFECT OF THE INVENTION]

The compound represented by Formula (I), used in the present invention, has an excellent effect of being effective in the treatment of pain. The compound represented by Formula (I) also has an excellent effect of being effective in the treatment of interstitial cystitis and/or bladder pain syndrome.

[BRIEF DESCRIPTION OF DRAWING]

[Fig. 1] Fig. 1 shows effects on the voiding interval in an acetic acid-induced urination disorder model. Rates of changes in the voiding interval after administration of the compound represented by Formula (I) are shown, with the value (Pre) of the voiding interval before the administration being taken as 100%.

[Fig. 2] Fig. 2 shows effects on the amount of voided urine per urinating action in an acetic acid-induced urination disorder model. Rates of changes in the amount of voided urine per urinating action after administration of the compound represented by Formula (I) are shown, with the value (Pre) of the amount of voided urine per urinating action before the administration being taken as 100%.

[MODE FOR CARRYING OUT THE INVENTION]

The term "consist of" means that a constituent element(s) is/are included exclusively. The term "comprise" means that an element(s) included is/are not limited to a constituent element(s) described, and an undescribed element(s) is/are not excluded.

Hereinafter, the present invention will be described with reference to embodiments. Throughout the present specification, an expression in the singular form should be understood as also encompassing the concept of its plural form, unless otherwise stated. Therefore, the singular articles (for example, "a", "an", "the", and the like in English) should be understood as also encompassing the concept of their plural form, unless otherwise stated.

In addition, the terms used in the present specification should be understood as being used in the meanings commonly used in the art unless otherwise stated. Therefore, unless otherwise defined, all terminology and scientific terms used in the present specification have the same meanings as commonly understood by those skilled in the art to which the present invention belongs. In case of conflict, the present specification (including definitions) shall prevail.

A pharmaceutical composition of the present invention for treating, alleviating, and/or preventing neuropathic pain, nociceptive pain, or visceral pain, as well as a pharmaceutical composition of the present invention for treating, alleviating, and/or preventing interstitial cystitis and/or bladder pain syndrome, are characterized by being a pharmaceutical composition comprising, as an active ingredient, a compound represented by Formula (I):

[Chemical Formula 10]

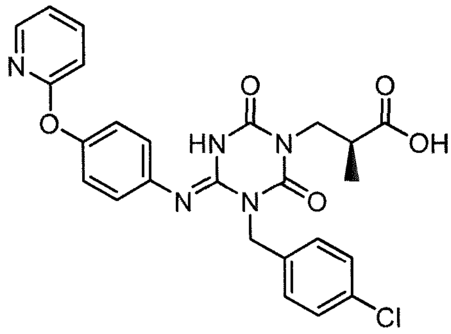

(I)

or a pharmaceutically acceptable salt thereof. Note that in the present specification, the pharmaceutical composition of the present invention for treating, alleviating, and/or preventing neuropathic pain, nociceptive pain, or visceral pain is also referred to as a therapeutic, palliative, and/or prophylactic agent for neuropathic pain, a therapeutic, palliative, and/or prophylactic agent for nociceptive pain, or a therapeutic, palliative, and/or prophylactic agent for visceral pain. In addition, pharmaceutical composition of the present invention for treating, alleviating, and/or preventing interstitial cystitis and/or bladder pain syndrome is also referred to as a therapeutic, palliative, and/or prophylactic agent for interstitial cystitis and/or bladder pain syndrome.

The compound represented by Formula (I) or a pharmaceutically acceptable salt thereof used in the present invention may be a solvate thereof.

The compound represented by Formula (I) is (2S)-3-(3-[(4-chlorophenyl)methyl]-2,6-dioxo-4-{[4-(pyridin-2-yloxy)phenyl]amino}-3,6-dihydro-1,3,5-triazin-1(2H)-yl)-2-methyl-propanoic acid, and has $P2X_3$ and/or $P2X_{2/3}$ receptor antagonist activity. In addition, the compound represented by Formula (I) includes the following tautomers:

[Chemical Formula 11]

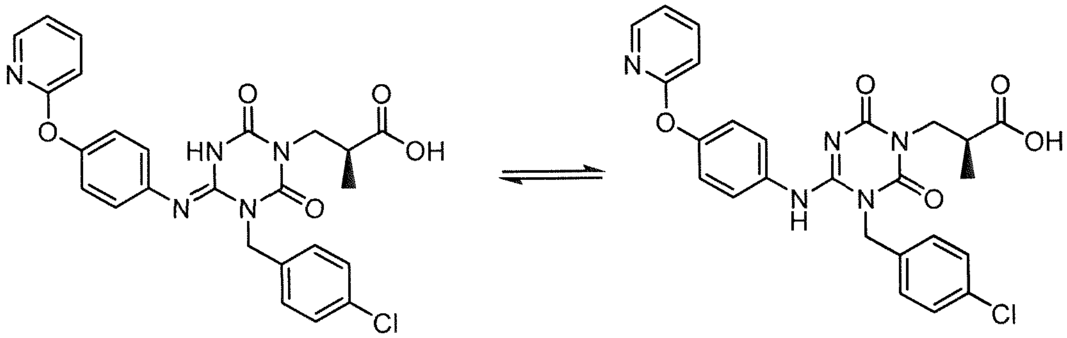

(I) (I)

The compound represented by Formula (I) can be synthesized according to a known method, for example, the methods described in International Publication No. WO 2014/200078 and International Publication No. WO 2012/020749.

In the present description, as the "pharmaceutically acceptable salt", examples of basic salts include alkali metal salts such as lithium salt, sodium salt and potassium salt; alkaline earth metal salts such as calcium salt and barium salt; transition metal salts such as zinc salt and iron salt; magnesium salt; ammonium salt; aliphatic amine salts such as trimethylamine salt, triethylamine salt, dicyclohexylamine salt, ethanolamine salt, diethanolamine salt, triethanolamine salt, ethylenediamine salt, meglumine salt and procaine salt; aralkylamine salts such as N,N-dibenzylethylenediamine; heterocyclic aromatic amine salts such as pyridine salt, picoline salt, quinoline salt and isoquinoline salt; quaternary ammonium salts such as tetramethylammonium salt, tetraethylammonium salt, benzyltrimethylammonium salt, benzyltriethylammonium salt, benzyltributylammonium salt, methyltrioctylammonium salt and tetrabutylammonium

salt; and basic amino acid salts such as arginine salt and lysine salt. Examples of acidic salts include inorganic acid salts such as hydrochloride, sulfate, nitrate, phosphate, carbonate, hydrogen carbonate, hydrobromate, hydroiodide and perchlorate; organic acid salts such as formate, acetate, propionate, trifluoroacetate, citrate, lactate, tartrate, oxalate, maleate, fumarate, mandelate, glutarate, malate, benzoate, phthalate and ascorbate; sulfonates such as methanesulfonate, ethanesulfonate, isethionate, benzenesulfonate and p-toluenesulfonate; and acidic amino acid salts such as aspartate and glutamate.

Solvates include organic solvates in which any number of organic solvent molecules are coordinated and hydrates in which any number of water molecules are coordinated. In the present description, the term "solvate" means a solvate of the compound represented by the above Formula (I) or a pharmaceutically acceptable salt thereof, and examples thereof include a monosolvate, a disolvate, a monohydrate and a dihydrate.

Pharmaceutically acceptable salts and solvates can be synthesized according to a known method.

In addition, as other pharmaceutical raw materials, additives such as excipients, binders, disintegrants, lubricants, sweeteners, flavoring agents, preservatives, chelating agents, antioxidants, cooling agents, coating agents, stabilizers, fluidizers, viscous agents, solubilizers, thickeners, buffers, flavors, colorants, adsorbents, wetting agents, moisture-proof agents, antistatic agents, plasticizers, antifoaming agents, surfactants, and emulsifiers may be contained. Specifically, binders (for example, corn starch, and the like), fillers (for example, lactose, microcrystalline cellulose, and the like), disintegrants (for example, sodium starch glycolate, and the like), and lubricants (for example, magnesium stearate, and the like) can be mentioned. Their contents are not limited.

The pharmaceutical composition for the treatment of chronic cough of the present invention can be prepared according to a method known to those skilled in the art. Moreover, the shape and size of the therapeutic agent are not limited. However, oral preparations are preferable, and among these, solid preparations are more preferable. Examples of dosage forms of solid preparations can include tablets (including orally fast disintegrating tablets, chewable tablets, effervescent tablets, jelly drops, and the like), lozenges, granules, pills, powders (including fine granules), and capsules (including hard capsules and soft capsules). Moreover, when preparing these, a coating treatment may be performed by a known method.

The pharmaceutical composition of the present invention can be administered orally or parenterally. Examples of the parenteral administration method include percutaneous administration, subcutaneous administration, intravenous administration, intraarterial administration, intramuscular administration, intraperitoneal administration, transmucosal administration, inhalation, transnasal administration, eye drops, ear drops, and intravaginal administration.

For oral administration, any dosage form usually used such as a solid preparation for internal use (e.g., tablets, powders, granules, capsules, pills, and films) or a liquid preparation for internal use (e.g., suspensions, emulsions, elixirs, syrups, lemonades, spirits, aromatic water, extracts, decoctions, and tinctures) can be prepared according to a routine method and administered. The tablets may be sugar-coated tablets, film-coated tablets, enteric coated tablets, sustained-release tablets, troche tablets, sublingual tablets, buccal tablets, chewable tablets or orally disintegrating tablets. The powders and the granules may be dry syrups. The capsules may be soft capsules, microcapsules or sustained-release capsules.

For parenteral administration, any dosage form usually used such as an injection, drops, and an external preparation (e.g., eye drops, nasal drops, ear drops, aerosols, inhalants, lotions, injectable fillers, liniments, gargles, enemas, ointments, plasters, gels, creams, patches, poultices, powders for external use, and suppositories) can be suitably administered. The injection may be an emulsion of O/W, W/O, O/W/O, W/O/W type, or the like.

The dose of the ingredients when the pharmaceutical composition of the present invention is used as a pharmaceutical composition for treating, alleviating, and/or preventing neuropathic pain, nociceptive pain or visceral pain, as well as the dose of the ingredients when the pharmaceutical composition of the present invention is used as a pharmaceutical composition for treating, alleviating, and/or preventing interstitial cystitis and/or bladder pain syndrome differ depending on the mode of administration, patient symptoms, age, weight, gender, or other concomitant drugs (if any), and is ultimately left to the discretion of the physician. Examples thereof include an aspect in which a daily adult dose of 10 to 450 mg of the compound represented by Formula (I) or a pharmaceutically acceptable salt thereof is orally administered.

In one embodiment, a daily adult dose of 10 mg to 300 mg of the compound represented by Formula (1) or a pharmaceutically acceptable salt thereof is orally administered.

In one embodiment, a daily adult dose of 20 mg to 300 mg of the compound represented by Formula (I) or a pharmaceutically acceptable salt thereof is orally administered.

In one embodiment, a daily adult dose of 30 mg to 300 mg of the compound represented by Formula (I) or a pharmaceutically acceptable salt thereof is orally administered.

In one embodiment, a daily adult dose of 50 mg to 300 mg of the compound represented by Formula (I) or a pharmaceutically acceptable salt thereof is orally administered.

In one embodiment, a daily adult dose of 70 mg to 300 mg of the compound represented by Formula (I) or a pharmaceutically acceptable salt thereof is orally administered.

In one embodiment, a daily adult dose of 100 mg to 300 mg of the compound represented by Formula (I) or a pharmaceutically acceptable salt thereof is orally administered.
In one embodiment, a daily adult dose of 150 mg to 300 mg of the compound represented by Formula (I) or a pharmaceutically acceptable salt thereof is orally administered.
In one embodiment, a daily adult dose of 200 mg to 300 mg of the compound represented by Formula (I) or a pharmaceutically acceptable salt thereof is orally administered.
In one embodiment, a daily adult dose of 10 mg to 200 mg of the compound represented by Formula (I) or a pharmaceutically acceptable salt thereof is orally administered.
In one embodiment, a daily adult dose of 20 mg to 200 mg of the compound represented by Formula (I) or a pharmaceutically acceptable salt thereof is orally administered.
In one embodiment, a daily adult dose of 30 mg to 200 mg of the compound represented by Formula (I) or a pharmaceutically acceptable salt thereof is orally administered.
In one embodiment, a daily adult dose of 50 mg to 200 mg of the compound represented by Formula (I) or a pharmaceutically acceptable salt thereof is orally administered.
In one embodiment, a daily adult dose of 70 mg to 200 mg of the compound represented by Formula (I) or a pharmaceutically acceptable salt thereof is orally administered.
In one embodiment, a daily adult dose of 100 mg to 200 mg of the compound represented by Formula (I) or a pharmaceutically acceptable salt thereof is orally administered.
In one embodiment, a daily adult dose of 150 mg to 200 mg of the compound represented by Formula (I) or a pharmaceutically acceptable salt thereof is orally administered.
In one embodiment, a daily adult dose of 10 mg to 150 mg of the compound represented by Formula (I) or a pharmaceutically acceptable salt thereof is orally administered.
In one embodiment, a daily adult dose of 20 mg to 150 mg of the compound represented by Formula (I) or a pharmaceutically acceptable salt thereof is orally administered.
In one embodiment, a daily adult dose of 30 mg to 150 mg of the compound represented by Formula (I) or a pharmaceutically acceptable salt thereof is orally administered.
In one embodiment, a daily adult dose of 50 mg to 150 mg of the compound represented by Formula (I) or a pharmaceutically acceptable salt thereof is orally administered.
In one embodiment, a daily adult dose of 70 mg to 150 mg of the compound represented by Formula (I) or a pharmaceutically acceptable salt thereof is orally administered.
In one embodiment, a daily adult dose of 100 mg to 150 mg of the compound represented by Formula (I) or a pharmaceutically acceptable salt thereof is orally administered.
In one embodiment, a daily adult dose of, for example, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150mg, 155mg, 160mg, 165mg, 170mg, 175mg, 180mg, 185mg, 190mg, 195mg, and 200mg of the compound represented by Formula (I) or a pharmaceutically acceptable salt thereof is orally administered.
In one embodiment, a daily adult dose of, for example, 50mg, 100mg, 150mg, 200mg, 250mg, or 300mg of the compound represented by Formula (I) or a pharmaceutically acceptable salt thereof is orally administered.
The dose may be administered at once or in divided doses. For example, the above dose is administered once a day. For example, the above dose is administered in two divided doses per day. For example, the above dose is administered in three divided doses per day. For example, the above dose is administered in four divided doses per day.

[EXAMPLES]

Hereinafter, the present invention will be described based on examples. However, the present invention is not limited to these examples and the like.

(Test Example 1) Evaluation of human $P2X_3$ receptor inhibitory activity

A stably expressing cell line in which C6BU-1 cells were transfected with human $P2X_3$ receptor gene (GenBank accession number Y07683) was seeded on a PDL-coated 96-well microplate so as to have 8000 cells per well, and then, cultured in medium (DMEM containing 7.0% fetal bovine serum, 7.0% horse serum, 1% antibiotic-antimycotic mixed stock solution, 4.0 mM glutamine) at 37°C and in 5% carbon dioxide for 1 day. The medium was replaced with a solution containing 4 μM of Fluo-3-AM (20 mM HEPES, 137 mM NaCl, 5.37 mM KCl, 0.9 mM $MgCl_2$, 1.26 mM $CaCl_2$, 5.6 mM D-glucose, 2.5 mM probenecid, 0.5% BSA, 0.04% Pluronic (registered trademark) F-127, pH 7.5), and then, incubation was carried out at 37°C and in 5% carbon dioxide for 1 hour. The wells were washed with wash buffer (20 mM HEPES, 137 mM NaCl, 5.27 mM KCl, 0.9 mM $MgCl_2$, 1.26 mM $CaCl_2$, 5.6 mM D-glucose, 2.5 mM probenecid, pH

7.5) and the microplate was filled with 40 μL of wash buffer per well. The microplate was placed in the high-throughput screening system FDSS 7000EX (Hamamatsu Photonics). The measurement of fluorescence intensity by FDSS 7000EX was started, and 40 μL per well of a DMSO solution of the compound represented by Formula (I) diluted with a dilution buffer (20 mM HEPES, 137 mM NaCl, 5.27 mM KCl, 0.9 mM $MgCl_2$, 1.26 mM $CaCl_2$, 5.6 mM D-glucose, 2.5 mM probenecid, 0.1% Pluronic (registered trademark) F-127, pH 7.5) to different concentrations was each dispensed by the automatic pipetting device built in the FDSS 7000EX. After 5 minutes, 50 μL of ATP solution diluted with the dilution buffer (final concentration of 50 nM) was dispensed by the automatic pipetting device built in the FDSS 7000EX, and then the measurement of the fluorescence intensity was continued for 4 minutes. From the measured fluorescence intensity values, the specific maximum fluorescence intensity, which is expressed as the ratio of the maximum fluorescence intensity value after addition of the ATP solution to the fluorescence intensity at the start of measurement, was calculated for each microplate well. With the value of the specific maximum fluorescence intensity when the compound represented by Formula (I) was not contained as 0% inhibition, and the value of the specific maximum fluorescence intensity when the dilution buffer was added instead of ATP as 100% inhibition, the concentration for 50% inhibition ($IC_{50}$) was calculated to evaluate the inhibitory activity of the compound represented by Formula (I). The specific maximum fluorescence intensity was calculated using the FDSS software (Hamamatsu Photonics). The $IC_{50}$ was calculated using the software Microsoft Excel (Microsoft) and XLfit (idbs).

(Results)

The $IC_{50}$ was 0.004 μM.

(Test Example 2) Evaluation of human $P2X_3$ receptor inhibitory activity in the presence of human serum albumin (HSA)

A stably expressing cell line in which C6BU-1 cells have been transfected with human $P2X_3$ receptor gene (GenBank accession number Y07683) is seeded on a PDL-coated 96-well microplate so as to have 8000 cells per well, and then, cultured in medium (DMEM containing 7.0% fetal bovine serum, 7.0% horse serum, 1% antibiotic-antimycotic mixed stock solution, 2.0% glutamine) at 37°C and in 5% carbon dioxide for 1 day. The medium is replaced with a solution containing 4 μM of Fluo-3-AM (20 mM HEPES, 137 mM NaCl, 5.37 mM KCl, 0.9 mM $MgCl_2$, 1.26 mM $CaCl_2$, 5.6 mM D-glucose, 2.5 mM probenecid, 0.5% BSA, 0.04% Pluronic F-127, pH 7.5), and then, incubation is carried out at 37°C and in 5% carbon dioxide for 1 hour. The wells are washed with wash buffer (20 mM HEPES, 137 mM NaCl, 5.27 mM KCl, 0.9 mM $MgCl_2$, 1.26 mM $CaCl_2$, 5.6 mM D-glucose, 2.5 mM probenecid, pH 7.5) and the microplate is filled with 40 μL of wash buffer per well. The microplate is placed in the high-throughput screening system FDSS 7000EX (Hamamatsu Photonics). The measurement of fluorescence intensity by FDSS 7000EX is started, and 40 μL per well of a DMSO solution of the compound represented by Formula (I) diluted to different concentrations with a solution of a dilution buffer (20 mM HEPES, 137 mM NaCl, 5.27 mM KCl, 0.9 mM $MgCl_2$, 1.26 mM $CaCl_2$, 5.6 mM D-glucose, 2.5 mM probenecid, 0.1% Pluronic (registered trademark) F-127, pH 7.5) to which human serum albumin is added to a final concentration of 1%, is each dispensed by the automatic pipetting device built in the FDSS 7000EX. After 5 minutes, 50 μL of ATP solution diluted with the dilution buffer (final concentration of 50 nM) is dispensed by the automatic pipetting device built in the FDSS 7000EX, and then the measurement of the fluorescence intensity is continued for 4 minutes. From the measured fluorescence intensity values, the specific maximum fluorescence intensity, which is expressed as the ratio of the maximum fluorescence intensity value after addition of the ATP solution to the fluorescence intensity at the start of measurement, is calculated for each microplate well. With the value of the specific maximum fluorescence intensity when the compound represented by Formula (I) is not contained as 0% inhibition, and the value of the specific maximum fluorescence intensity when the dilution buffer is added instead of ATP as 100% inhibition, the concentration for 50% inhibition ($IC_{50}$) is calculated to evaluate the inhibitory activity of the compound represented by Formula (I). The specific maximum fluorescence intensity is calculated using the FDSS software (Hamamatsu Photonics). The $IC_{50}$ is calculated using the software Microsoft Excel (Microsoft) and XLfit (idbs).

(Test Example 3) Evaluation of rat $P2X_3$ receptor inhibitory activity

Stably expressing cells in which C6BU-1 cells have been transfected with rat $P2X_3$ receptor gene (GenBank accession number NM_031075) are seeded on a PDL-coated 96-well microplate so as to have 8000 cells per well, then cultured in medium (DMEM containing 7.0% fetal bovine serum, 7.0% horse serum, 1% antibiotic-antimycotic mixed stock solution, 4.0 mM glutamine) at 37°C and in 5% carbon dioxide for 1 day. The medium is replaced with a solution containing 4 μM of Fluo-3-AM (20 mM HEPES, 137 mM NaCl, 5.37 mM KCl, 0.9 mM $MgCl_2$, 1.26 mM $CaCl_2$, 5.6 mM D-glucose, 2.5 mM probenecid, 0.5% BSA, 0.04% Pluronic F-127, pH 7.5), and then, incubation is carried out at 37°C and in 5% carbon dioxide for 1 hour. The wells are washed with wash buffer (20 mM HEPES, 137 mM NaCl, 5.27 mM KCl, 0.9 mM $MgCl_2$, 1.26 mM $CaCl_2$, 5.6 mM D-glucose, 2.5 mM probenecid, pH 7.5) and the microplate is filled with

40 μL of wash buffer per well. The microplate is placed in the high-throughput screening system FDSS 7000EX (Hamamatsu Photonics). The measurement of fluorescence intensity by FDSS 7000EX is started, and 40 μL per well of a DMSO solution of the compound represented by Formula (I) diluted with a dilution buffer (20 mM HEPES, 137 mM NaCl, 5.27 mM KCl, 0.9 mM $MgCl_2$, 1.26 mM $CaCl_2$, 5.6 mM D-glucose, 2.5 mM probenecid, 0.1% Pluronic (registered trademark) F-127, pH 7.5) to different concentrations is each dispensed by the automatic pipetting device built in the FDSS 7000EX. After 5 minutes, 50 μL of ATP solution diluted with the dilution buffer (final concentration of 50 nM) is dispensed by the automatic pipetting device built in the FDSS 7000EX, and then the measurement of the fluorescence intensity is continued for 4 minutes. From the measured fluorescence intensity values, the specific maximum fluorescence intensity, which is expressed as the ratio of the maximum fluorescence intensity value after addition of the ATP solution to the fluorescence intensity at the start of measurement, is calculated for each microplate well. With the value of the specific maximum fluorescence intensity when the compound represented by Formula (I) is not contained as 0% inhibition, and the value of the specific maximum fluorescence intensity when the dilution buffer is added instead of ATP as 100% inhibition, the concentration for 50% inhibition ($IC_{50}$) is calculated to evaluate the inhibitory activity of the compound represented by Formula (I). The specific maximum fluorescence intensity is calculated using the FDSS software (Hamamatsu Photonics). The $IC_{50}$ is calculated using the software Microsoft Excel (Microsoft) and XLfit (idbs).

(Test Example 4) Evaluation of rat $P2X_3$ receptor inhibitory activity in the presence of rat serum albumin (RSA)

A stably expressing cell line in which C6BU-1 cells have been transfected with human $P2X_3$ receptor gene (GenBank accession number NM_031075) was seeded so that 8000 cells were present per well, and then, cultured in medium (DMEM containing 7.0% fetal bovine serum, 7.0% horse serum, 1% antibiotic-antimycotic mixed stock solution) at 37°C and in 5% carbon dioxide for 1 day. The medium was replaced with a solution containing 4 μM of Fluo-4-AM (20 mM HEPES, 137 mM NaCl, 5.27 mM KCl, 0.9 mM $MgCl_2$, 1.26 mM $CaCl_2$, 5.6 mM D-glucose, 2.5 mM probenecid, 10% BSA, 0.08% Pluronic (registered trademark) F-127, pH 7.5), and then, incubation was carried out at 37°C and in 5% carbon dioxide for 1 hour. The wells were washed with wash buffer (20 mM HEPES, 137 mM NaCl, 5.27 mM KCl, 0.9 mM $MgCl_2$, 1.26 mM $CaCl_2$, 5.6 mM D-glucose, 2.5 mM probenecid, pH 7.5) and the microplate was filled with 40 μL of wash buffer per well. The microplate was placed in the high-throughput screening system FDSS 7000EX (Hamamatsu Photonics). The measurement of fluorescence intensity by FDSS 7000EX was started, and 40 μL per well of a DMSO solution of the compound represented by Formula (I), diluted to different concentrations with a solution of a dilution buffer (20 mM HEPES, 137 mM NaCl, 5.27 mM KCl, 0.9 mM $MgCl_2$, 1.26 mM $CaCl_2$, 5.6 mM D-glucose, 2.5 mM probenecid, 0.1% Pluronic F-127, pH 7.5) to which rat serum albumin was added to a final concentration of 1%, was each dispensed by the automatic pipetting device built in the FDSS 7000EX. After 5 minutes, 50 μL of 50 nM ATP solution diluted with the dilution buffer was dispensed by the automatic pipetting device built in the FDSS 7000EX, and then the measurement of the fluorescence intensity was continued for 4 minutes. From the measured fluorescence intensity values, the specific maximum fluorescence intensity, which is expressed as the ratio of the maximum fluorescence intensity value after addition of the ATP solution to the fluorescence intensity at the start of measurement, was calculated for each microplate well. With the value of the specific maximum fluorescence intensity when the compound represented by Formula (I) was not contained as 0% inhibition, and the value of the specific maximum fluorescence intensity when the dilution buffer was added instead of ATP as 100% inhibition, the concentration for 50% inhibition ($IC_{50}$) was calculated to evaluate the inhibitory activity of the compound represented by Formula (I). The specific maximum fluorescence intensity was calculated using the FDSS software (Hamamatsu Photonics). The $IC_{50}$ was calculated using the software Microsoft Excel (Microsoft) and XLfit (idbs).

(Results)

The $IC_{50}$ was 10 nM.

(Test Example 5) Assessment of drug efficacy in Seltzer model

Partial sciatic nerve ligation model in rats

Preparation of model

Each rat was anesthetized with isoflurane and the hair of the left thigh was shaved. An incision was made in the skin just below the hip bone. The muscle was bluntly dissected to expose the sciatic nerve. 1/3 to 1/2 of the sciatic nerve was strongly ligated with a thread, and the muscle and the skin were sutured. This is referred to as a nerve ligated side. The right thigh underwent an identical procedure with the left hind limb except for the ligation of the sciatic nerve. This is referred to as a sham operated side.

Evaluation (1)

Two weeks after the operation, the effect on tactile allodynia was assessed using von Frey filaments. Two weeks after the operation, for habituation, the rats were placed into a plastic cage on a wire mesh bottom. Von Frey filaments (0.4 to 26 g) were applied to the plantar surface of the rat hind paws from the wire mesh side, and the value of the von Frey filament pressure at which the paw was withdrawn was used as a pain threshold. Pain thresholds were measured for the left and right hind limbs and these were used as pre-treatment pain thresholds. The rats showing the pain threshold of 0.6 to 2 g on the nerve ligated side and showing the threshold of 8 to 15 g on the sham operated side were employed in the experiments. Before the measurement of the pre-treatment pain thresholds, the rats had their hind paws set on the apparatus to familiarize them with the test procedure. The compound represented by Formula (I) was administered to the employed rats. The compound represented by Formula (I) was homogenized with mortar and pestle and suspended or diluted in 0.5% Methyl Cellulose to prepare a suspension, and it was orally administered at 10 mg/kg to the rats using a syringe attached with a sonde. Pain thresholds were measured for the right and left hind limbs at 3 hours after the drug administration, and these were used as post-treatment pain thresholds. A percent reversal value for each rat was calculated using the following formula. The analgesic effects of the compound were compared.

$$\% \text{ Reversal value} = 100 \times [\text{Log}_{10}(\text{post-treatment pain threshold on nerve ligated side}) - \text{Log}_{10}(\text{pre-treatment pain threshold on nerve ligated side})] / [\text{Log}_{10}(\text{pre-treatment pain threshold on sham operated side}) - \text{Log}_{10}(\text{pre-treatment pain threshold in nerve ligated side})]$$

(Results)

A percent reversal value of 65% was indicated.

(Test Example 6) Assessment of drug efficacy in diabetic neuropathic pain model Preparation of rat diabetic neuropathic pain model

To overnight fasted rats, 60 mg of streptozotocin (STZ) was intravenously administered to prepare a diabetes model. As a control, rats to which saline was administered were also prepared.

Evaluation (1)

Two weeks after the administration of STZ, the analgesic effect of the compound represented by Formula (I) was evaluated by the Randall-Selitto method. In this test, a pressure stimulus was applied to the leg of the test animal, and the degree of the pressure stimulus was gradually increased to observe the response to pain, and the stimulus that caused the response was defined as a pain threshold. On the day of the test, the pain threshold before the administration of the compound represented by Formula (I) was measured, and then after the administration of the test substance, the pain threshold was measured. The compound represented by Formula (I) was homogenized with mortar and pestle and suspended in 0.5% Methyl Cellulose to prepare a suspension, and it was orally administered at 10 mg/kg to the animals using a syringe attached with a sonde. Pain thresholds were measured for the hind limbs at 3 hours after the administration of the test substance, and these were used as post-administration pain thresholds. A percent reversal value for each rat was calculated using the following formula. The analgesic effects of the compound were compared.

$$\% \text{ Reversal value} = (\text{post-administration pain threshold} - \text{pre-administration pain threshold}) / (\text{pain threshold of saline-administered rat} - \text{pre-administration pain threshold}) \times 100$$

(Results)

A percent reversal value of 55% was indicated.

(Test Example 7) Assessment of drug efficacy in knee osteoarthritis pain model Preparation of rat knee osteoarthritis model

Under isoflurane anesthesia, a knee joint on one side was shaved, and a monoiode sodium acetate (MIA)

solution (saline solution, up to 5 mg/50 μL/rat) was intraarticularly administered.

Evaluation (1)

Two weeks after the MIA administration, the analgesic effect of the compound represented by Formula (I) was evaluated. This test was based on a system for measuring a load distribution applied to hind limbs of a rat, and a ratio of load differences between left and right hind limbs was used as a pain threshold. On the day of the test, the pain threshold before the administration of the compound represented by Formula (I) was measured, and then after the administration of the test substance, the pain threshold was measured. The compound represented by Formula (I) was homogenized with mortar and pestle and suspended in 0.5% Methyl Cellulose to prepare a suspension, and it was orally administered at 10 mg/kg to the animals using a syringe attached with a sonde. Pain thresholds were measured for the hind limbs at 3 hours after the administration of the test substance, and these were used as post-administration pain thresholds. The load difference was measured up to three times, and the average value thereof was used as the pain threshold.

A percent reversal value for each rat was calculated using the following formula. The analgesic effects of the compound were compared.

% Reversal value = (post-administration pain threshold - pre-administration pain threshold) / (pain threshold of saline-administered rat - pre-administration pain threshold) × 100

(Results)

A percent reversal value of 51% was indicated.

(Test Example 8) Assessment of drug efficacy in inflammatory pain model Preparation of rat inflammatory pain model

Under isoflurane anesthesia, FCA (12.5 -100 μg) was subcutaneously injected into the hind limb of a rat to prepare an inflammatory pain model.

Evaluation (1)

Two weeks after the administration of FCA, the analgesic effect of the compound represented by Formula (I) was evaluated by the Randall-Selitto method. In this test, a pressure stimulus was applied to the leg of the test animal, and the degree of the pressure stimulus was gradually increased to observe the response to pain, and the stimulus that caused the response was defined as a pain threshold. On the day of the test, the pain threshold before the administration of the compound represented by Formula (I) was measured, and then after the administration of the test substance, the pain threshold was measured. The compound represented by Formula (I) was homogenized with mortar and pestle and suspended in 0.5% Methyl Cellulose to prepare a suspension, and it was orally administered at 10 mg/kg to the animals using a syringe attached with a sonde. Pain thresholds were measured for the hind limbs at 1 hour after the administration of the test substance, and these were used as post-administration pain thresholds. A percent reversal value for each rat was calculated using the following formula. The analgesic effects of the compound were compared.

% Reversal value = (post-administration pain threshold - pre-administration pain threshold) / (pain threshold of hind limb on FCA-non-administered side - pre-administration pain threshold) × 100

(Results)

A percent reversal value of 58% was indicated.

(Test Example 9) Assessment of drug efficacy in cystitis pain model

Preparation of mouse bladder pain model

Cyclophosphamide was dissolved in physiological saline or the like, and the solution was intraperitoneally administered to mice. Cyclophosphamide was administered at 150 mg/kg once daily consecutively for four days. As a control, mice to which saline was administered were also prepared.

Evaluation (1)

The analgesic effect of the compound represented by Formula (I) was evaluated the day after the final administration of cyclophosphamide. In this test, mechanical stimulation by an automatic von Frey device was applied to the periphery of the lower abdomen while holding the mouse, and with respect to the resulting escape behavior defined as pain-like behavior, the pain threshold was determined. On the day of the test, the pain threshold before the administration (pre-administration pain threshold) of the compound represented by Formula (I) was measured, and then after the administration of the test substance, the pain threshold was measured. The compound represented by Formula (I) was homogenized with mortar and pestle and suspended or diluted in 0.5% Methyl Cellulose to prepare a suspension, and it was orally administered at 3 mg/kg to the rats using a syringe attached with a sonde. Pain thresholds were measured at 1 hour after the administration of the test substance, and these were used as post-administration pain thresholds. A percent reversal value for each rat was calculated using the following formula. The analgesic effects of the compound were compared.

% Reversal value = (post-administration pain threshold - pre-administration pain threshold) / (pain threshold of saline-administered mouse - pre-administration pain threshold) × 100

(Results)

A percent reversal value of 46% was indicated.

(Test Example 10) Assessment of drug efficacy in mouse bone cancer pain model Preparation of mouse bone cancer pain model

The skin was incised under isoflurane anesthesia, the muscles were torn to expose the left femur, a hole was made in the bone marrow cavity using a 26 G needle, and tumor cells (NCTC2472 cells derived from C3H/HeN mice) were transplanted using a 30 G needle. The hole made in the femoral bone marrow cavity was closed with dental cement, and then the skin was sutured. Mice in shamoperated groups were prepared by administering HBSS instead of tumor cells.

Evaluation (1)

Two weeks after the operation, the effect on tactile allodynia was assessed using von Frey filaments. Two weeks after the operation, for habituation, the mice were placed into a plastic cage on a wire mesh bottom. Von Frey filaments were applied to the plantar surface of the mouse hind paws from the wire mesh side, and the value of the von Frey filament pressure at which the paw was withdrawn was used as a pain threshold. The pain threshold was evaluated for the operated hind limb and defined as a pre-administration pain threshold, and the compound represented by the formula (I) was administered to the animal at 10 mg/kg. The compound represented by Formula (I) was homogenized with mortar and pestle and suspended or diluted in 0.5% Methyl Cellulose to prepare a suspension, and it was orally administered to the rats using a syringe attached with a sonde. Pain thresholds were measured for the operated hind limbs after the administration of the test substance, and these were used as post-administration pain thresholds. A percent reversal value for each rat was calculated using the following formula. The analgesic effects of the compound were compared.

% Reversal value = 100 x [$Log_{10}$(post-operation/administration pain threshold) - $Log_{10}$(pre-operation/administration pain threshold)] / [$Log_{10}$(pain threshold on sham operated side) - $Log_{10}$(pre-operation/administration pain threshold)]

(Results)

A percent reversal value of 55% was indicated.

(Test Example 11) Assessment of drug efficacy in acetic acid-induced urination disorder model

Preparation of acetic acid-induced urination disorder model

A catheter was placed in a rat bladder under isoflurane anesthesia. After 2 days from the surgery for placing the catheter, 0.3% acetic acid was administered at 4 mL/hr over 30 minutes to induce cystitis. In this model, the amount of urine per urinating action and the voiding interval decreased.

Evaluation (1)

On the day after acetic acid was administered, the amount of urine per urinating action and the voiding interval before drug administration were evaluated. The compound represented by Formula (I) was homogenized with mortar and pestle and suspended in 0.5% Methyl Cellulose to prepare a suspension, and it was orally administered at 0.1 mg/kg, 0.75 mg/kg, or 3 mg/kg to the rats using a syringe attached with a sonde. The amount of urine per urinating action and the voiding interval during a period from 30 minutes to 150 minutes after administration were measured. The value before administration for each individual was assumed to be 100%, and the change rate after administration was calculated.

Rate of change in amount of voided urine per urinating action
= (amount of urine per urinating action after drug treatment of cystitis rat ÷ amount of voided urine per urinating action before drug treatment of cystitis rat) × 100

Rate of change in voiding interval
= (voiding interval after drug treatment of cystitis rat ÷ voiding interval before drug treatment of cystitis rat) × 100

(Results)

Rate of change in amount of voided urine per urinating action: 208.4% (3 mg/kg administration group)

Rate of change in voiding interval: 219.0% (3 mg/kg administration group)

As shown in Figs. 1 and 2, a significant improving effect was shown by administration of 3 mg/kg of the compound represented by Formula (I).

All data are shown as mean ± SEM.

$: $p < 0.05$; significantly different from vehicle (Dunnett's multiple comparison test)

From the results of Test Example 5 to 10 described above, it was found that the compound represented by Formula (I), which is a $P2X_3$ and/or $P2X_{2/3}$ receptor antagonist, or a pharmaceutically acceptable salt thereof has a high improvement rate of pain and exhibits effectiveness in treating pain. In addition, from the results of Test Example 9 and Test Example 11, it was found that the composition was effective in treating cystitis pain, and further, it was found that the prolongation of the voiding interval and the associated increase in the amount of voided urine per urinating action were confirmed. Thus, it was found that the composition was effective in treating interstitial cystitis and/or bladder pain syndrome.

Formulation Example

The following Formulation Examples are only exemplified and not intended to limit the scope of the invention.

(Formulation Example 1) Suspension

For example, water for injection was added to the active pharmaceutical ingredient, the compound represented by Formula (I), to prepare a suspension.

(Formulation Example 2) Tablets

For example, lactose and magnesium stearate were added as additives to the active pharmaceutical ingredient, the compound represented by Formula (I), to prepare tablets.

[INDUSTRIAL APPLICABILITY]

A pharmaceutical composition comprising a compound represented by Formula (I) or a pharmaceutically acceptable salt thereof is useful as a medicament for treating, alleviating, and/or preventing neuropathic pain, nociceptive pain, or visceral pain. In particular, it is useful as a medicament for treating, alleviating, and/or preventing peripheral neuropathic pain, diabetic neuropathic pain, cystitis pain, or pain associated with endometriosis. Furthermore, it is useful as a medicament for treating, alleviating, and/or preventing interstitial cystitis and/or bladder pain syndrome.

## Claims

1. A pharmaceutical composition for treating, alleviating, and/or preventing neuropathic pain, nociceptive pain, or visceral pain, comprising a compound represented by Formula (I):

[Chemical Formula 1]

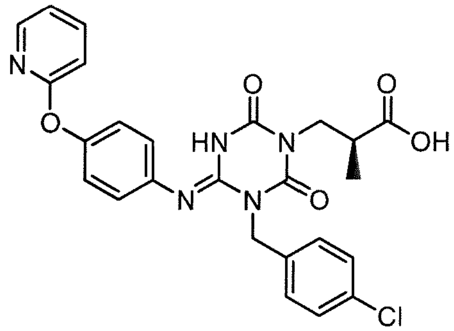

(I)

or a pharmaceutically acceptable salt thereof.

2. The pharmaceutical composition according to claim 1, wherein the neuropathic pain is peripheral neuropathic pain.

3. The pharmaceutical composition according to claim 1 or 2, wherein the neuropathic pain is diabetic neuropathic pain, postoperative neuropathic pain, or cancer pain.

4. The pharmaceutical composition according to any one of claims 1 to 3, wherein the neuropathic pain is diabetic neuropathic pain.

5. The pharmaceutical composition according to claim 1, wherein the nociceptive pain is inflammatory pain, knee osteoarthritis pain, or cancer pain.

6. The pharmaceutical composition according to claim 1, wherein the visceral pain is cystitis pain, cancer pain, or pain associated with endometriosis.

7. The pharmaceutical composition according to claim 1 or 6, wherein the visceral pain is cystitis pain.

8. A pharmaceutical composition for treating, alleviating, and/or preventing interstitial cystitis and/or bladder pain syndrome, comprising a compound represented by Formula (I):

[Chemical Formula 2]

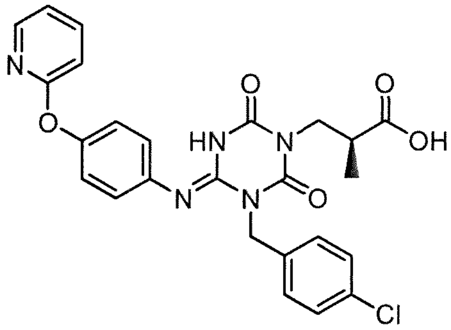

(I)

or a pharmaceutically acceptable salt thereof.

**9.** The pharmaceutical composition according to any one of claims 1 to 8, wherein a daily dose of an active ingredient of the pharmaceutical composition is 10 mg to 450 mg.

**10.** The pharmaceutical composition according to any one of claims 1 to 9, to be administered once daily.

**11.** A method for treating, alleviating, and/or preventing neuropathic pain, nociceptive pain, or visceral pain, the method comprising the step of administering an effective amount of a compound represented by Formula (I):

[Chemical Formula 3]

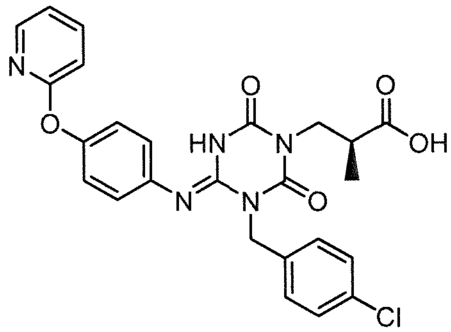

(I)

or a pharmaceutically acceptable salt thereof to an individual in need of treatment, alleviation, and/or prevention of neuropathic pain, nociceptive pain, or visceral pain.

**12.** The treating, alleviating, and/or preventing method according to claim 11, wherein the neuropathic pain is peripheral neuropathic pain.

**13.** The treating, alleviating, and/or preventing method according to claim 11 or 12, wherein the neuropathic pain is diabetic neuropathic pain, postoperative neuropathic pain, or cancer pain.

**14.** The treating, alleviating, and/or preventing method according to any one of claims 11 to 13, wherein the neuropathic pain is diabetic neuropathic pain.

**15.** The treating, alleviating, and/or preventing method according to claim 11, wherein the nociceptive pain is inflammatory pain, knee osteoarthritis pain, or cancer pain.

**16.** The treating, alleviating, and/or preventing method according to claim 11, wherein the visceral pain is cystitis pain, cancer pain, or pain associated with endometriosis.

**17.** The treating, alleviating, and/or preventing method according to claim 11 or 16, wherein the visceral pain is cystitis pain.

**18.** A method for treating, alleviating, and/or preventing interstitial cystitis and/or bladder pain syndrome, the method

comprising the step of administering an effective amount of a compound represented by Formula (I):

[Chemical Formula 4]

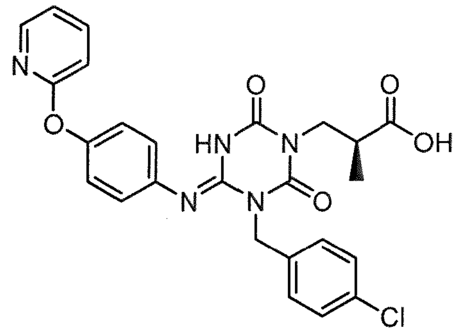

(I)

or a pharmaceutically acceptable salt thereof to an individual in need of treatment, alleviation, and/or prevention of interstitial cystitis and/or bladder pain syndrome.

**19.** The treating, alleviating, and/or preventing method according to any one of claims 11 to 18, wherein a daily dose of an active ingredient is 10 mg to 450 mg.

**20.** The treating, alleviating, and/or preventing method according to any one of claims 11 to 19, wherein the administering step is performed once daily.

**21.** Use of a compound represented by Formula (I):

[Chemical Formula 5]

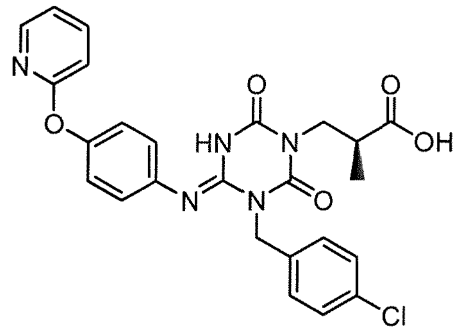

(I)

or a pharmaceutically acceptable salt thereof for manufacture of a medicament for treating, alleviating, and/or preventing neuropathic pain, nociceptive pain, or visceral pain.

**22.** The use according to claim 21, wherein the neuropathic pain is peripheral neuropathic pain.

**23.** The use according to claim 21 or 22, wherein the neuropathic pain is diabetic neuropathic pain, postoperative neuropathic pain, or cancer pain.

**24.** The use according to any one of claims 21 to 23, wherein the neuropathic pain is diabetic neuropathic pain.

**25.** The use according to claim 21, wherein the nociceptive pain is inflammatory pain, knee osteoarthritis pain, or cancer pain.

**26.** The use according to claim 21, wherein the visceral pain is cystitis pain, cancer pain, or pain associated with endometriosis.

**27.** The use according to claim 21 or 26, wherein the visceral pain is cystitis pain.

**28.** Use of a compound represented by Formula (I):

[Chemical Formula 6]

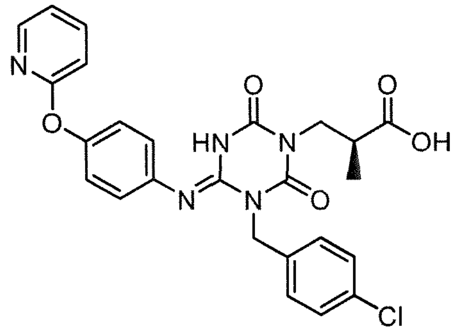

(I)

or a pharmaceutically acceptable salt thereof for manufacture of a medicament for treating, alleviating, and/or preventing interstitial cystitis and/or bladder pain syndrome.

**29.** The use according to any one of claims 21 to 28, wherein a daily dose of an active ingredient is 10 mg to 450 mg.

**30.** The use according to any one of claims 21 to 29, wherein the medicament is administered once daily.

Fig. 1

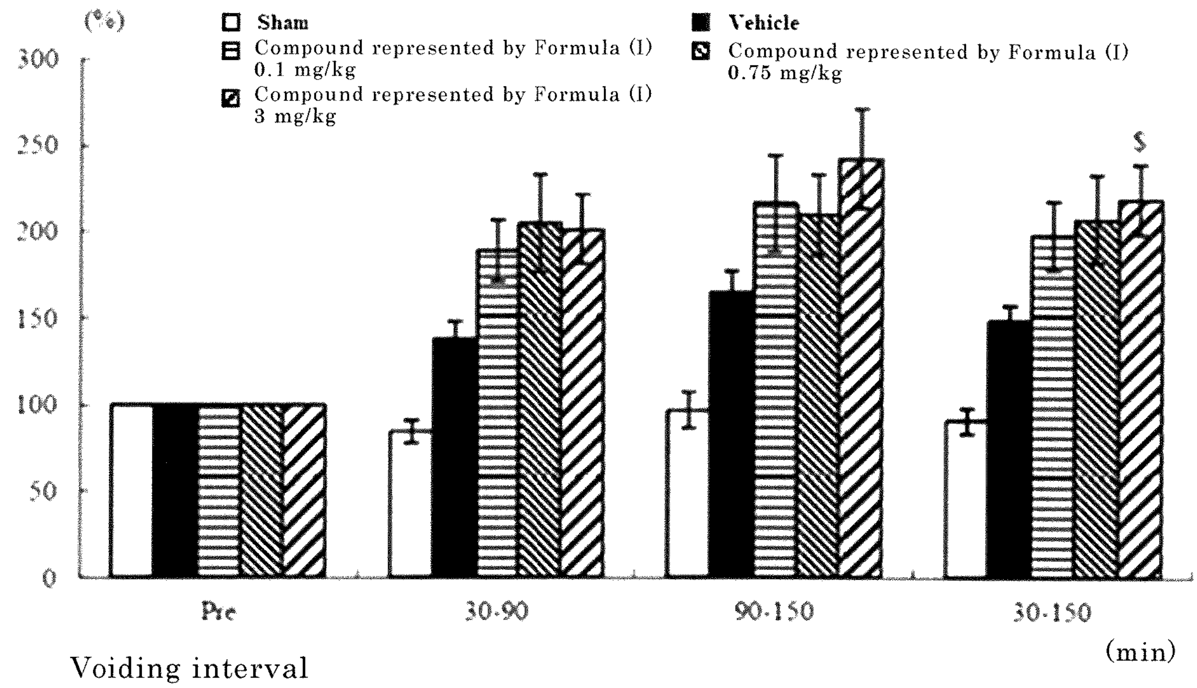


Voiding interval

Fig. 2

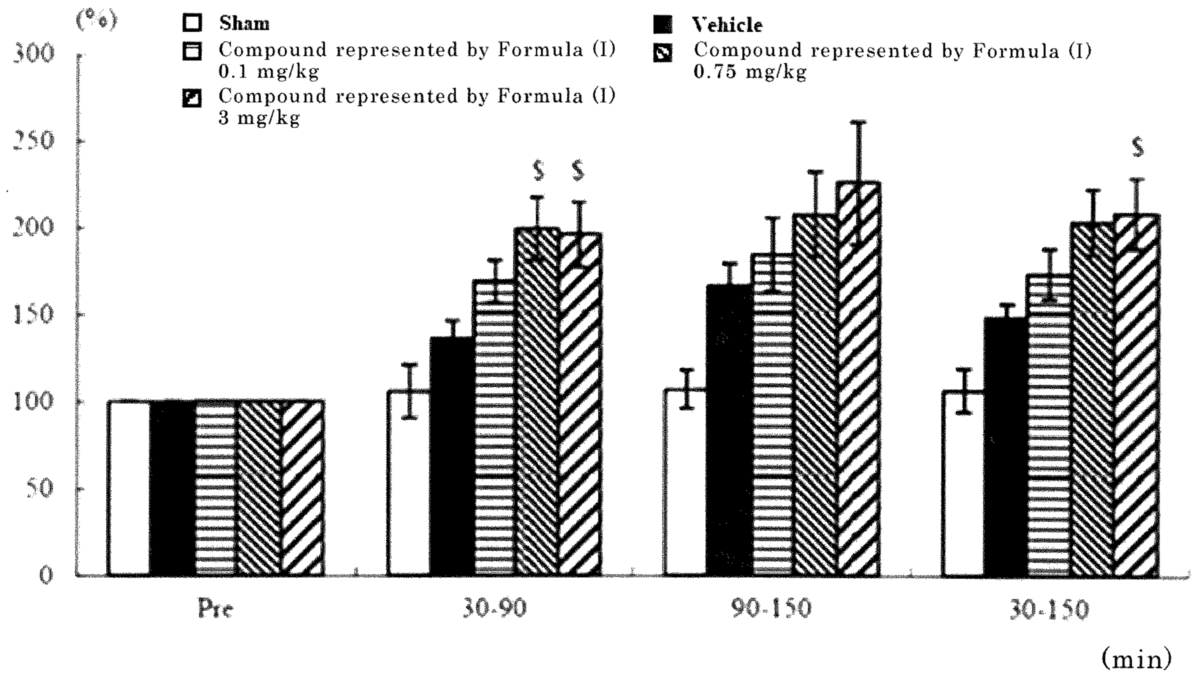


Amount of voided urine per urinating action

**INTERNATIONAL SEARCH REPORT**

International application No.
PCT/JP2021/014261

A. CLASSIFICATION OF SUBJECT MATTER
A61K 31/53(2006.01)i; A61P 25/04(2006.01)i; A61P 29/02(2006.01)i; A61P 43/00(2006.01)i
FI: A61K31/53; A61P25/04; A61P29/02; A61P43/00 111
According to International Patent Classification (IPC) or to both national classification and IPC

B. FIELDS SEARCHED

Minimum documentation searched (classification system followed by classification symbols)
A61K31/53; A61P25/04; A61P29/02; A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/REGISTRY/MEDLINE/EMBASE/BIOSIS (STN), JSTPlus/ JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| Y | WO 2014/200078 A1 (SHIONOGI & CO., LTD.) 18 December 2014 (2014-12-18) entire text, in particular, claims, paragraph [0117], compound no. 1-127, test examples | 1-30 |
| Y | WO 2018/169286 A1 (GWANGJU INSTITUTE OF SCIENCE AND TECHNOLOGY) 20 September 2018 (2018-09-20) entire text, in particular, claims, experiment examples | 1-30 |
| Y | YUAN, Ming et al., "Effect of A-317491 delivered by glycolipid-like polymer micelles on endometriosis pain", International Journal of Nanomedicine, 2017, vol. 12, pp. 8171-8183, ISSN: 1178-2013 entire text, in particular, Abstract, Conclusion | 1-30 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 April 2021 (23.04.2021) | 11 May 2021 (11.05.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2021/014261

C (Continuation). DOCUMENTS CONSIDERED TO BE RELEVANT

| Category* | Citation of document, with indication, where appropriate, of the relevant passages | Relevant to claim No. |
|---|---|---|
| Y | BURNSTOCK, G., "Targeting the visceral purinergic system for pain control", Current Opinion in Pharmacology, 2012, vol. 12, pp. 80-86, ISSN 1471-4892 entire text, in particular, Abstract, Introduction, Potential therapeutic strategies | 1-30 |
| Y | ZHANG, Huiping et al., "The function of P2X3 receptor and NK1 receptor antagonists on cyclophosphamide-induced cystitis in rats", World Journal of Urology, 2014, vol. 32, pp. 91-97, ISSN0724-4983 entire text, in particular, Abstract, Introduction, Comment | 1-30 |
| A | WO 2012/020749 A1 (SHIONOGI & CO., LTD.) 16 February 2012 (2012-02-16) entire text | 1-30 |
| A | WO 2013/089212 A1 (SHIONOGI & CO., LTD.) 20 June 2013 (2013-06-20) entire text | 1-30 |
| A | WO 2010/092966 A1 (SHIONOGI & CO., LTD.) 19 August 2010 (2010-08-19) entire text | 1-30 |

Form PCT/ISA/210 (continuation of second sheet) (January 2015)

**INTERNATIONAL SEARCH REPORT**
Information on patent family members

International application No.
PCT/JP2021/014261

| Patent Documents referred in the Report | Publication Date | Patent Family | Publication Date |
|---|---|---|---|
| WO 2014/200078 A1 | 18 Dec. 2014 | EP 3009432 A1<br>entire text, in particular, claims, paragraph [0174], compound no. I-127, test examples<br>US 2016/0115151 A1<br>US 2017/0362199 A1<br>AU 2014279116 A<br>CA 2915325 A<br>KR 10-2016-0018807 A<br>CN 105452234 A<br>EA 201690019 A<br>MX 2015016877 A<br>KR 10-2017-0116177 A<br>BR 112015031079 A<br>TW 201529567 A<br>AR 96620 A | |
| WO 2018/169286 A1 | 20 Sep. 2018 | JP 2020-514346 A<br>US 2020/0131131 A1<br>KR 10-2018-0104528 A<br>CN 110770209 A | |
| WO 2012/020749 A1 | 16 Feb. 2012 | US 2013/0172317 A1<br>entire text<br>EP 2604595 A1<br>AU 2011290261 A<br>CA 2807947 A<br>CN 103153968 A<br>MX 2013001542 A<br>KR 10-2013-0138734 A<br>RU 2013110068 A<br>BR 112013002984 A<br>TW 201210600 A<br>TW 201542210 A | |
| WO 2013/089212 A1 | 20 Jun. 2013 | TW 201331188 A | |
| WO 2010/092966 A1 | 19 Aug. 2020 | EP 2399910 A1<br>entire text<br>US 2011/0319414 A1<br>US 2016/0185736 A1<br>CA 2752269 A<br>KR 10-2011-013470 A<br>AU 2010214356 A<br>SG 173647 A<br>MX 2011008362 A<br>CN 102395571 A<br>RU 2011137531 A<br>TW 201033183 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02094767 A
- WO 2010092966 A
- WO 2012020749 A
- WO 2013089212 A
- WO 2014200078 A
- WO 2020071530 A


### Non-patent literature cited in the description


- *J. Physiology,* 2003, vol. 554 (2), 301-308
- *J. Physiology,* 2003, vol. 553 (3), 683-694
- *Pflungers Arch Eur J physiol,* 2006, vol. 452, 513-537
- *Neuroscientist,* 2005, vol. 11, 345-356
- *Mol Neurobiol,* 2007, vol. 36, 165-83
- *Expert Opin.Ther.Patens,* 2006, vol. 16 (8), 1113-1127
- *PNAS,* 2002, vol. 99 (26), 17179-17184
- *Purinergic Signal.,* 2012, vol. 8, 3-26
- *Am J Physiol Gastrointest Liver Physiol,* 2015, vol. 308, 710-719
- *PLoS ONE,* 2017, vol. 12 (9
- *International Journal of Nanomedicine,* 2017, vol. 12, 8171-8183